# EUROPEAN PATENT SPECIFICATION

(11) **EP 3 036 258 B1**
(45) Date of publication and mention of the grant of the patent: **19.07.2023**
(21) Application number: 14838641.0
(22) Date of filing: 22.08.2014
(51) Int. Cl.: C07K 16/28, A61K 39/395, A61P 37/04, A61P 35/00, A61P 31/12

(54) **IMMUNORECEPTOR MODULATION FOR TREATING CANCER AND VIRAL INFECTIONS**
IMMUNREZEPTORMODULATION ZUR BEHANDLUNG VON KREBS UND VIRUSINFEKTIONEN
MODULATION D'IMMUNORÉCEPTEUR DESTINÉE AU TRAITEMENT DE CANCER ET D'INFECTIONS VIRALES

(30) Priority: 22.08.2013 AU 2013903189; 03.10.2013 WO PCT/AU2013/001132; 05.03.2014 AU 2014900741; 21.03.2014 AU 2014901002
(43) Date of publication of application: 29.06.2016
(73) Proprietor: The Council of the Queensland Institute of Medical Research, Herston, Queensland 4006 (AU)
(72) Inventor: SMYTH, Mark, Herston, Queensland 4006 (AU)
(74) Representative: Schiweck Weinzierl Koch Patentanwälte Partnerschaft mbB
(86) International application number: PCT/AU2014/000830
(87) International publication number: WO 2015/024060

(56) References cited:
- WO-A1-2005/107799
- WO-A1-2011/057216
- WO-A2-2004/074324
- WO-A2-2008/073316
- WO-A2-2009/126688
- CN-A- 102 204 901
- CN-A- 102 204 901
- A. FUCHS ET AL: "Cutting Edge: CD96 (Tactile) Promotes NK Cell-Target Cell Adhesion by Interacting with the Poliovirus Receptor (CD155)", THE JOURNAL OF IMMUNOLOGY, vol. 172, no. 7, 19 March 2004 (2004-03-19), pages 3994-3998, XP055344481, US ISSN: 0022-1767, DOI: 10.4049/jimmunol.172.7.3994
- S A QUEZADA ET AL: "Exploiting CTLA-4, PD-1 and PD-L1 to reactivate the host immune response against cancer", BRITISH JOURNAL OF CANCER, vol. 108, no. 8, 19 March 2013 (2013-03-19), pages 1560-1565, XP055344904, GB ISSN: 0007-0920, DOI: 10.1038/bjc.2013.117
- D. MEYER ET AL: "CD96 Interaction with CD155 via Its First Ig-like Domain Is Modulated by Alternative Splicing or Mutations in Distal Ig-like Domains", JOURNAL OF BIOLOGICAL CHEMISTRY, vol. 284, no. 4, 23 January 2009 (2009-01-23), pages 2235-2244, XP055345087, US ISSN: 0021-9258, DOI: 10.1074/jbc.M807698200
- S. J. BLAKE ET AL: "Suppression of Metastases Using a New Lymphocyte Checkpoint Target for Cancer Immunotherapy", CANCER DISCOVERY, vol. 6, no. 4, 1 April 2016 (2016-04-01), pages 446-459, XP055344484, US ISSN: 2159-8274, DOI: 10.1158/2159-8290.CD-15-0944
- CHRISTOPHER J CHAN ET AL: "The receptors CD96 and CD226 oppose each other in the regulation of natural killer cell functions", NATURE IMMUNOLOGY, vol. 15, no. 5, 23 March 2014 (2014-03-23) , pages 431-438, XP055344476, ISSN: 1529-2908, DOI: 10.1038/ni.2850
- MOHSENI NODEHI, S. ET AL.: 'Enhanced ADCC activity of affinity maturated and Fc- engineered mini-antibodies directed against the AML stem cell antigen CD 96.' PLOS ONE. vol. 7, no. 8, 2012, page E42426, XP055284783
- GRAMATZKI, M. ET AL.: 'Antibodies TC-12 (''unique'') and TH -111 ( CD 96) characterize T- cell acute lymphoblastic leukemia and a subgroup of acute myeloid leukemia.' EXP HEMATOL. vol. 26, no. 13, December 1998, pages 1209 - 14, XP008109027
- BARTH, B.M. ET AL.: 'Targeted indocyanine-green-loaded calcium phosptiosilicate nanoparticles for in vivo photodynamic therapy of leukemia.' ACS NANO. vol. 5, no. 7, 26 July 2011, pages 5325 - 37, XP055284785
- ZENG, J.M. ET AL.: 'Human CD 96 gene cloning, expression and identification''.' NAN FANG YI KE DA XUE XUE BAO. vol. 31, no. 7, June 2011, pages 1232 - 5, XP008182842
- GONG, J. ET AL.: 'Establishment of an enzyme-linked immunosorbent assay system for determining soluble CD 96 and its application in the measurement of sCD96 in patients with viral hepatitis B and hepatic cirrhosis.' CLIN EXP IMMUNOL. vol. 155, no. 2, February 2009, pages 207 - 15, XP055284787
- FUCHS, A. ET AL.: 'Cutting edge: CD 96 (tactile) promotes NK cell -target cell adhesion by interacting with the poliovirus receptor ( CD 155).' J IMMUNOL. vol. 172, no. 7, 01 April 2004, pages 3994 - 8, XP055284790
- SETH, S. ET AL.: 'The murine pan T cell marker CD 96 is an adhesion receptor for CD 155 and nectin-1.' BIOCHEM BIOPHYS RES COMMUN. vol. 364, no. 4, 28 December 2007, pages 959 - 65, XP022344218
- LARSEN, H. ET AL.: 'Nonviral transfection of leukemic primary cells and cells lines by siRNA-a direct comparison between Nucleofection and Accell delivery.' E XP HEMATOL. vol. 39, no. 11, November 2011, pages 1081 - 1089, XP028318425
- CHAN, C.J.: 'Receptors that interact with nectin and nectin-like proteins in the immunosurveillance and immunotherapy of cancer.' CURR OPIN IMMUNOL. vol. 24, no. 2, April 2012, pages 246 - 51, XP055147483
- STANIETSKY, N ET AL.: 'Paired NK cell receptors controlling NK cytotoxicity.' FEBS LETT vol. 584, no. 24, 15 December 2010, pages 4895 - 900, XP055284792
- FUCHS, A ET AL.: 'The role of NK cell recognition of nectin and nectin-like proteins in tumor immunosurveillance.' SEMIN CANCER BIOL. vol. 16, no. 5, October 2006, pages 359 - 66, XP024908054

## Description

### TECHNICAL FIELD

THIS INVENTION relates to the immunoreceptor CD96. More particularly, this invention relates to inhibition of CD96 to thereby enhance the ability of the immune system to target tumours and other diseases or conditions that can evade the immune system.

### BACKGROUND

The progression of a productive immune response requires that a number of immunological checkpoints be passed. Passage may require the presence of excitatory co-stimulatory signals or the avoidance of negative or co-inhibitory signals, which act to dampen or terminate immune activity. The immunoglobulin superfamily occupies a central importance in this coordination of immune responses, and the CD28/cytotoxic T-lymphocyte antigen-4 (CTLA-4):B7.1/B7.2 receptor/ligand grouping represents the archetypal example of these immune regulators. In part the role of these checkpoints is to guard against the possibility of unwanted and harmful self-directed activities. While this is a necessary function, aiding in the prevention of autoimmunity, it may act as a barrier to successful immunotherapies aimed at targeting malignant self-cells that largely display the same array of surface molecules as the cells from which they derive. Therapies aimed at overcoming these mechanisms of peripheral tolerance, in particular by blocking the inhibitory checkpoints on T cells, offer the potential to generate antitumor activity, either as monotherapies or in synergism with other therapies that directly or indirectly enhance presentation of tumor epitopes to the immune system. Such anti-T cell checkpoint antibodies are showing promise in early clinical trials of advanced human cancers.

Furthermore, natural killer (NK) cells are innate lymphocytes critical to limit early tumor growth and metastasis ¹. NK cell functions are also regulated by the integration of signals transmitted by a wide range of activating and inhibitory receptors ². For example, the recognition of pathogen-derived or stress-induced ligands by activating receptors such as NCRs, NKG2D, or DNAM-1 stimulate NK cells cytotoxicity and the secretion of pro-inflammatory mediators such as interferon gamma (IFN-γ) ³. In contrast, inhibitory receptors protect target cells from NK cell-mediated killing ⁴. These receptors mostly recognize MHC class I and MHC class I-related molecules and include the KIR (killer cell immunoglobulin-like receptors) and LIR (leukocyte immunoglobulin-like receptors) families, the Ly49 family in mice and the CD94/NKG2 heterodimers in both species.

An emerging group of immunoglobulin superfamily members that interact with ligands of the nectin and nectin-like (necl) family has recently been described to influence NK cell and T cell functions ⁵. These include CD226 (DNAM-1) ⁶, CD96 (TACTILE) 7, TIGIT (T cell immunoglobulin and ITIM domain) ^{8,9}, and CRTAM (class I restricted T cell-associated molecule) ¹⁰. DNAM-1 and TIGIT are the most extensively studied members of this family and they share a common ligand, CD155 (necl-5; PVR) and CD112 (nectin-2; PVRL2) ^{8,11}. TIGIT also bind an additional ligand CD113 (PVRL3) ⁸. The functions of DNAM-1 and TIGIT on NK cells are reportedly counter-balancing ¹². *In vitro,* DNAM-1 potentiates the cytotoxicity of NK cells against a wide range of tumor cells ^{13,14} and is critical for tumor immunosurveillance *in vivo* ^{13,15,16}. In contrast, TIGIT bear an ITIM motif and has been proposed prevent self-tissue damage similar to inhibitory Ly49 or KIR interactions with MHC class I ¹⁷. Indeed, engagement of TIGIT by CD155 has been shown to limit IFNγ production and cytotoxicity by NK cells *in vitro ^{18,19}.* However, the role of TIGIT in NK cell biology relative to the other nectin receptors DNAM-1 and CD96 remains to be assessed *in vivo.*

Despite being cloned 20 years ago ⁷, little is known about CD96, the other Ig family member that shares CD155 ligand with DNAM-1 and TIGIT ^{20,21}. In humans, CD96 expression is largely confined to NK cells, CD8 T cells, and CD4 T cells ⁷. The major ligand of CD96 is CD155, but CD96 has also been reported to associate with CD111 (nectin-1) and play a role in promoting NK and T cell adhesion ^{21,22}.

### SUMMARY

Surprisingly, the present inventors have discovered that CD96 acts as a negative regulator of T cell and NK cell anti-tumor functions. Accordingly, the invention is broadly directed to use of agents that at least partly block or inhibit CD96 to thereby reduce or relieve CD96-mediated immune inhibition to enhance or restore immune surveillance in the mammal. In certain embodiments, this may facilitate treatment of diseases or conditions responsive at least partial blocking or inhibition of CD96, such as cancers and/or viral infections.

In a first aspect, the invention provides a method of reducing or relieving immune inhibition in a mammal, said method including the step of at least partly inhibiting or reducing CD96 activity in one or more cells of the mammal to thereby relieve immune inhibition and/or enhance or restore immune surveillance in the mammal.

Suitably, the step of inhibiting or reducing CD96 activity in the mammal does not include, or at least depend upon, killing of CD96-expressing cells in the mammal. In some embodiments, the step of inhibiting or reducing CD96 activity in the mammal includes inhibiting or reducing CD96 binding to CD155 and/or intracellular signaling in one or more cells of the mammal that express CD96. In some embodiments, the step of inhibiting or reducing CD96 activity in the mammal includes removing cell surface CD96 and/or down-regulating cell surface expression of CD96.

In one particular embodiment, the step of inhibiting or reducing CD96 activity in the mammal includes increasing or enhancing expression, production and/or secretion of one or more cytokines or chemokines. Preferably, the cytokine is interferon γ (IFN-γ). Typically, the one or more cells of the mammal are T cells, inclusive of CD4⁺ and CD8⁺ T cells, γδT cells, NKT cells, and natural killer (NK) cells.

In a preferred embodiment, the method relieves immune inhibition and/or enhances or restores immune surveillance in the mammal to thereby treat or prevent cancer or cancer metastasis in the mammal.

In other embodiments, the method relieves immune inhibition and/or enhances or restores immune surveillance in the mammal to thereby treat or prevent a viral infection in the mammal.

In a second aspect, the invention provides a method of screening, designing, engineering or otherwise producing a CD96-inhibitory agent, said method including the step of determining whether a candidate molecule is capable of at least partly inhibiting or reducing CD96 activity to thereby relieve immune inhibition and/or enhance or restore immune surveillance in a mammal.

In a third aspect, the invention provides a CD96-inhibitory agent screened, designed, engineered or otherwise produced according to the method of the second aspect.

In one embodiment, the CD96-inhibitory agent is an antibody or antibody fragment.

In one particular embodiment, the CD96-inhibitory agent is an anti-cancer agent.

In another particular embodiment, the CD96-inhibitory agent is an anti-viral agent.

In a fourth aspect, the invention provides a CD96-inhibitory agent according to the third aspect for use according to the method of the first aspect.

Suitably, according to the aforementioned aspects the mammal is a human.

Any reference in the description to methods of treatment or in vivo diagnosis refer to the compounds, pharmaceutical compositions and medicaments of the present invention for use in method of treatment of the human or animal body by therapy or for in vivo diagnosis. Unless the context requires otherwise, the terms *"comprise", "comprises"* and *"comprising",* or similar terms are intended to mean a non-exclusive inclusion, such that a recited list of elements or features does not include those stated or listed elements solely, but may include other elements or features that are not listed or stated.

The indefinite articles 'a' and *'an'* are used here to refer to or encompass singular or plural elements or features and should not be taken as meaning or defining "one" or a "single" element or feature.

### BRIEF DESCRIPTION OF THE FIGURES

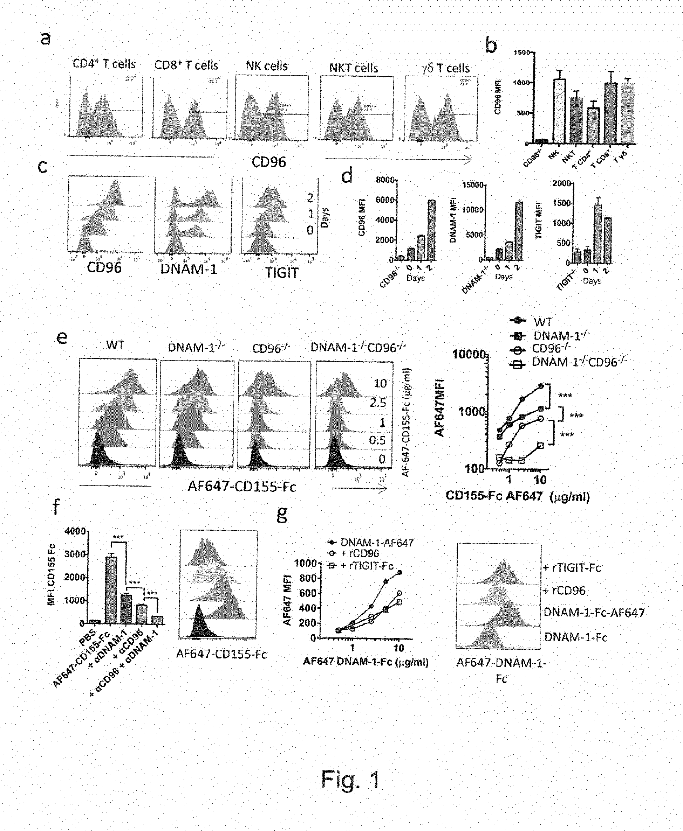

Figure 1: CD96 competes with DNAM-1 for CD155 binding. **a, b** The expression of CD96 was analyzed by flow cytometry on the indicated spleen lymphocyte populations from C57BL/6 WT (light grey) and *CD96*^{*-*/*-*} mice (dark grey). The representative FACS Histograms (**a**) and the mean ± SD (**b**) of 3 mice from one representative experiment out of 3 are shown. **c, d** The expression of CD96, DNAM-1 and TIGIT was determined on WT spleen NK cells freshly isolated or activated for 48 hrs with IL-2 (1000 U/ml). **e.** The binding of mouse CD155-Fc coupled with AF-647 to purified NK cells freshly isolated from WT, *CD96*^{*-*/}*⁻ DNAM-1*^{*-*/*-*} or *DNAM-1*^{*-*/}*⁻CD96*^{*-*/*-*} mice was assessed at the indicated concentrations by flow cytometry. **f.** The binding of CD155-Fc coupled with AF-647 (10 µg/ml) was analyzed on purified WT NK cells in the presence of anti-CD96 and or anti-DNAM-1 mAbs. **g.** The binding of DNAM-1-Fc labeled with AF-647 (0.5-10 µg/ml) at the cell surface of BMDC was analyzed in the presence of 50 µg/ml of control Ig, recombinant CD96 or TIGIT-Fc. **c-g**. The representative FACS Histograms and the mean ± SD of triplicate wells from one representative experiment out of at least 3 experiments are shown. *** p<0.001 Student T test.

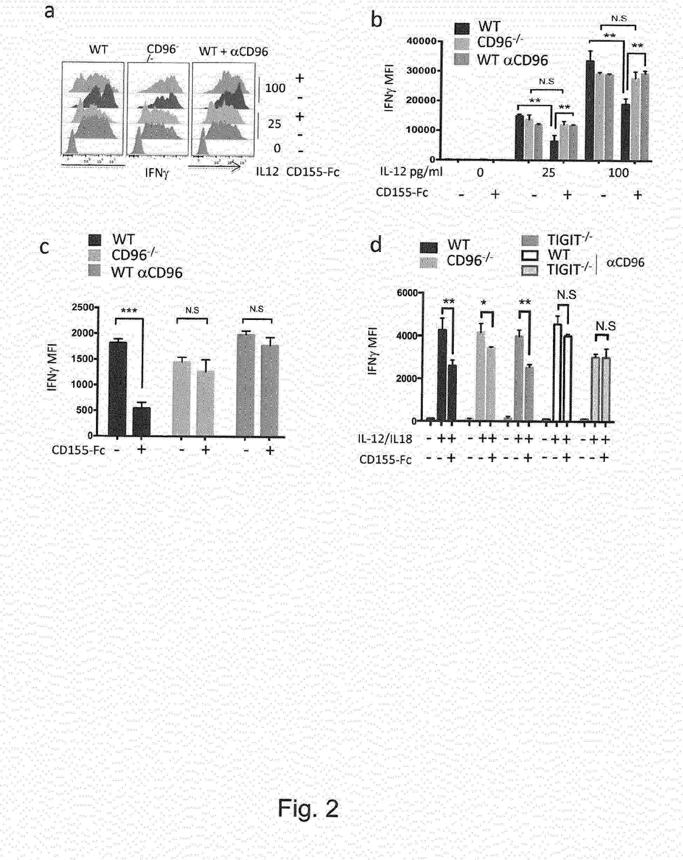

Figure 2: CD96 engagement by CD155 regulate NK cell production of IFNγ. CD96 binding to CD155-Fc limits the production of IFN-γ by NK cells induced by exogenous cytokines (**a, b, d)** and NK cell receptors (**c**). **a, b, d**. We analyzed the intracellular production of IFN-γ by freshly purified *CD96*^{*-*/}*⁻, TIGIT*^{*-*/*-*} and *WT* NK cells in the presence or absence of anti-CD96 (50 µg/ml) in response to IL-12 (25-100 pg/ml) and IL-18 (50 ng/ml) using plates coated with or without CD155-Fc (0.5 µg/ well). **c.** We analyzed the intracellular production of IFN-γ by IL-2-activated NK cells from *CD96*^{*-*/*-*} and *WT* mice using plates coated with anti-NK1.1 (2.5 µg/ well) and CD155-Fc (0.5 µg/ well). The representative FACS Histograms (**a**) and the mean ± SD of triplicate wells (**b, c, d**) from one representative experiment out of 3 are shown. *p <0.05, ** p <0.01, *** p<0.001, Student T test.

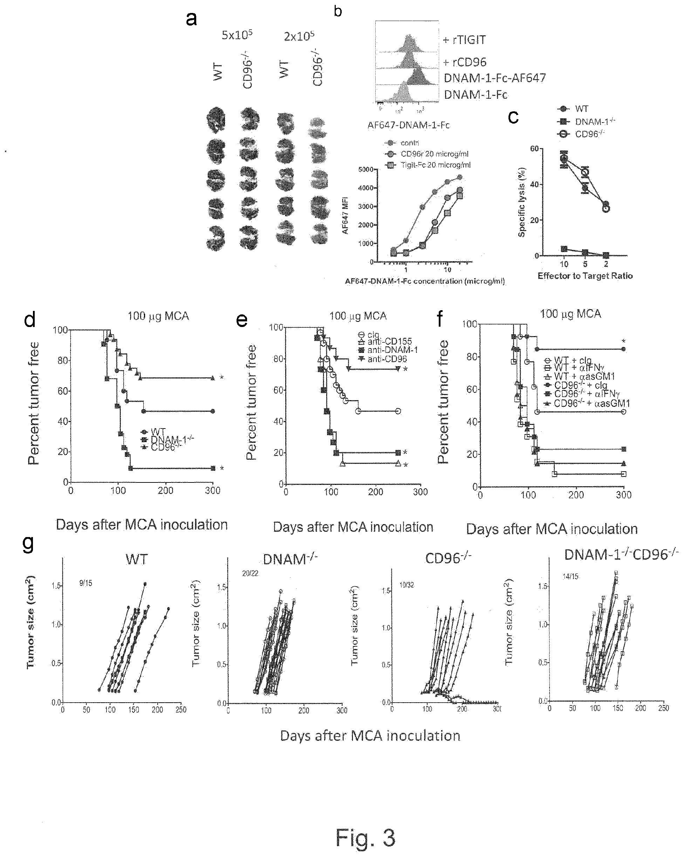

Figure 3: CD96 limits NK cell-dependent tumor immunosurveillance. **a.** CD96 and DNAM-1 have an opposite role in the control of B16F10 metastasis. Pictures showing the lung of WT and CD96^{-/-} mice two weeks after the injection of 2 × 10⁵ and 5 × 10⁵ B16F10 cells. Representative experiment out of two. **b.** CD96 and TIGIT compete with DNAM-1 for the binding of CD155 at the cell surface of B16F10. The binding of DNAM-1-Fc labeled with AF-647 (0.5-20 µg/ml) at the cell surface of B16F10 cells was analyzed in the presence of 50 µg/ml of control Ig, recombinant CD96 or TIGIT-Fc. The FACS histograms and the mean ± SD of triplicate wells from one representative experiment out of 3 are shown. **c.** A 4 hr ⁵¹Cr release assay was performed between B16F10 cells and IL-2-activated NK cells from WT, *DNAM-1*^{-/-} and *CD96*^{*-*/*-*} mice at the indicated effector target ratios. Solid circles represent WT NK cells, open circles represent CD96^{-/-} NK cells and solid squares represents DNAM-1^{-/-} NK cells, **e-h.** CD96 and DNAM-1 have an opposite role in the immunosurveillance of MCA induced fibrosarcoma mediated by NK cells, **d.-g.** Groups of 15-30 male, WT, *DNAM-1*^{*-*/*-*} and *CD96-1*^{*-*/*-*} and *DNAM-1*^{*-*/}*⁻CD96*^{*-*/*-*} mice were injected with MCA (100 µg/mouse). The survival (**d.-f.**) and the growth curves of individual mice with sarcoma (**g.**) are shown. **e.** WT mice were treated with an anti-CD96, anti-DNAM-1 or anti-CD155 mAbs as defined in the Materials and Methods, **f.** WT and *CD96*^{*-*/*-*} mice were injected with 100 µg MCA and treated with either a control antibody, anti-IFN-γ antibody, or anti-asialoGM1. * p<0.05 Mantel-Cox test.

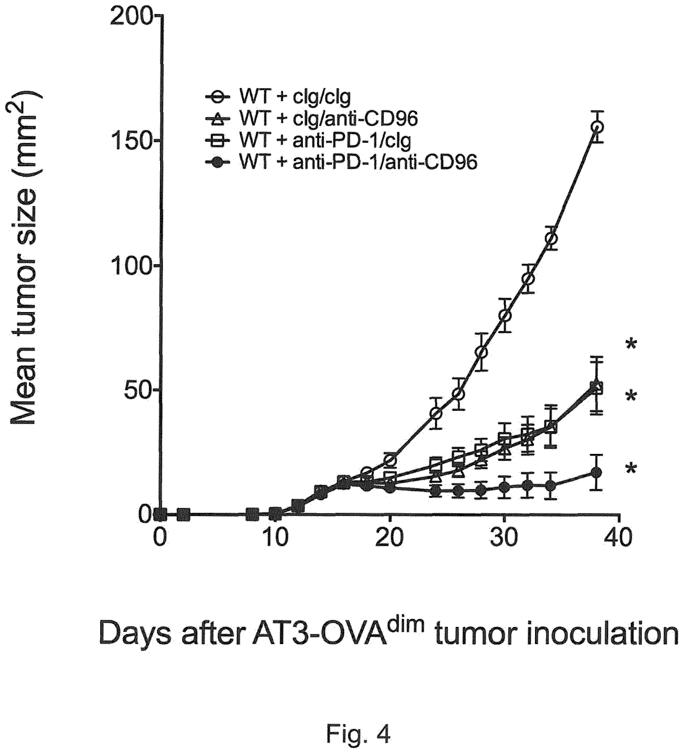

Figure 4: Anti-CD96 mAb has single agent activity and enhances the anti-tumor responses of anti-PD1. C57BL/6 wild type (WT) mice were injected subcutaneously with AT3-OVA^{dim} tumor cells (10⁶ cells) and treated on day 16, 20 and 24 with intraperitoneal injections of anti-CD96 mAb (3.3, 250 µg i.p) or anti-PD-1 (RMP1-14, 250 µg i.p.). Means ± SEM of 5 mice per group (mm²) are shown (*: p<0.05 compared to cIg alone by Mann-Whitney test).

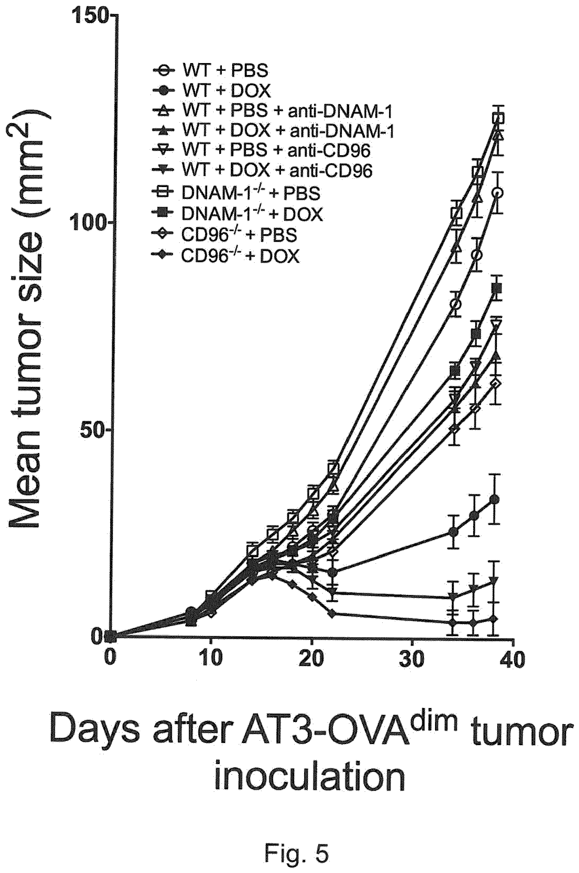

Figure 5: Anti-CD96 mAb enhances anti-tumor responses generated by Doxorubicin (DOX) chemotherapy. C57BL/6 wild type (WT), DNAM-1^{*-*/*-*}, and CD96^{-/-} mice were injected subcutaneously with AT3-OVA^{dim} tumor cells (10⁶ cells) and treated on day 14 with control PBS or DOX (50 microliters, 2 mM, intratumor). Some groups of WT mice also received on day 12, 14, 18, 21, 24 and 28 intraperitoneal injections of anti-CD96 mAb (3.3, 250 µg i.p) or anti-DNAM-1 (480.1, 250 µg i.p.). Means ± SEM of 5 mice per group (mm²) are shown.

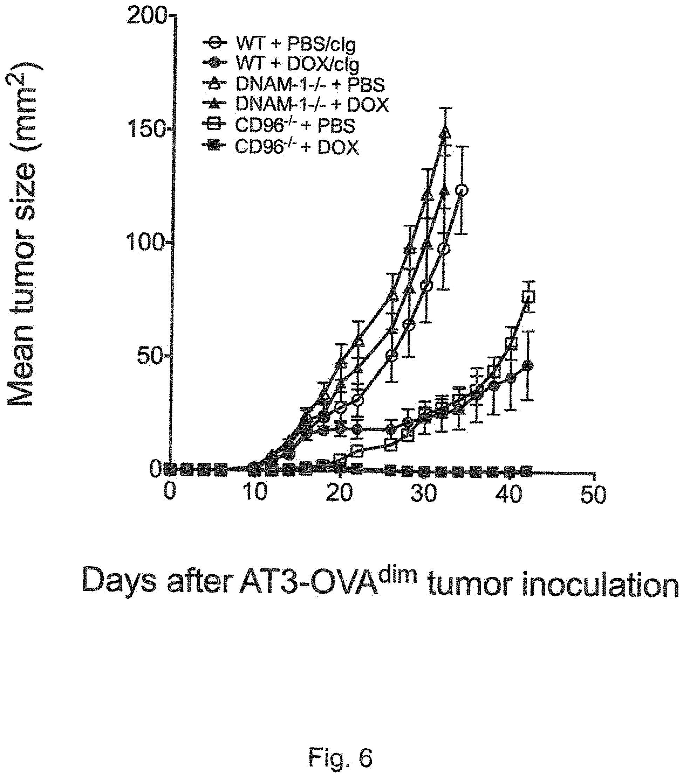

Figure 6: Enhanced anti-tumor responses of Doxorubicin (DOX) chemotherapy with host CD96 deficiency. C57BL/6 wild type (WT), DNAM-1^{*-*/*-*}, and CD96^{*-*/*-*} mice were injected subcutaneously with AT3-OVA^{dim} tumor cells (10⁶ cells) and treated on day 16 with control PBS or DOX (50 microliters, 2 mM, intratumor). Means ± standard errors of 5 mice per group (mm²) are shown.

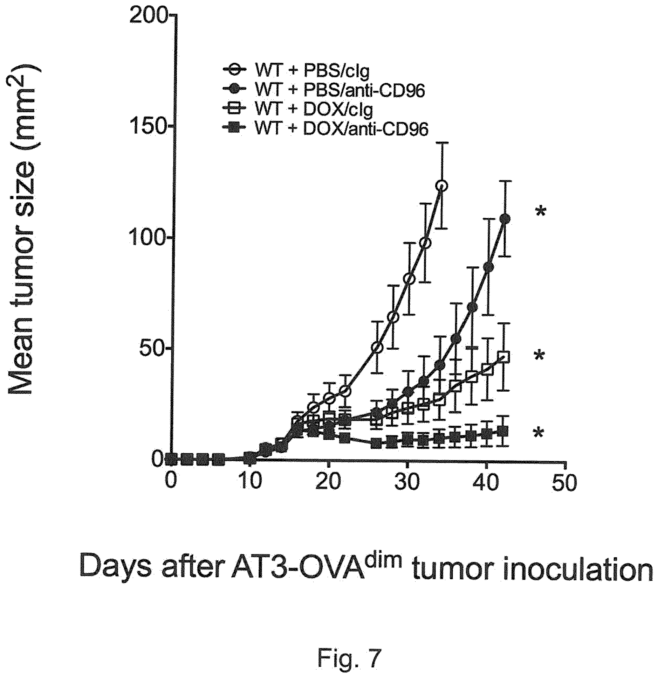

Figure 7: Anti-CD96 mAb enhances anti-tumor responses generated by Doxorubicin (DOX) chemotherapy. C57BL/6 wild type (WT) mice were injected subcutaneously with AT3-OVA^{dim} tumor cells (10⁶ cells) and treated on day 16 with control PBS or DOX (50 microliters, 2 mM, intratumor). Some groups of WT mice also received on day 16, 20, and 23 intraperitoneal injections of anti-CD96 mAb (3.3, 250 µg i.p). Means ± SEM of 5 mice per group (mm²) are shown (*: p<0.05 compared to cIg alone by Mann-Whitney test).

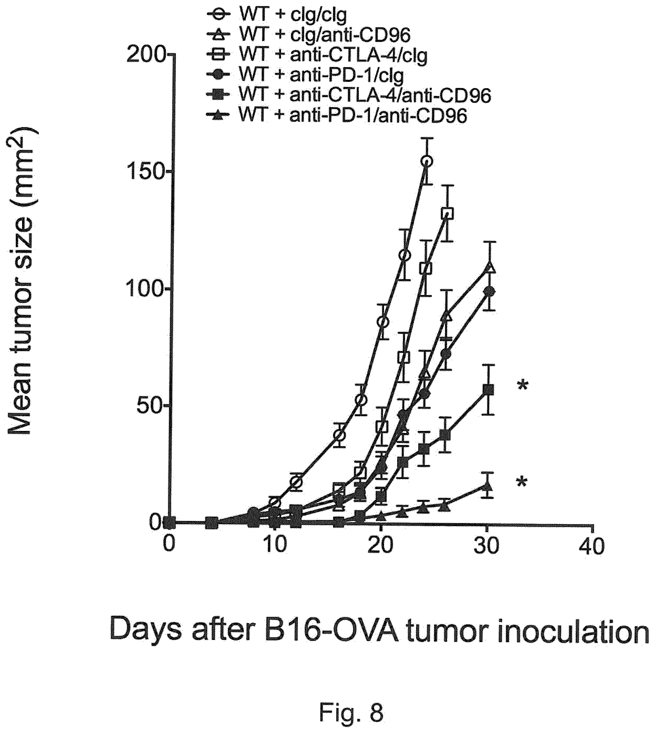

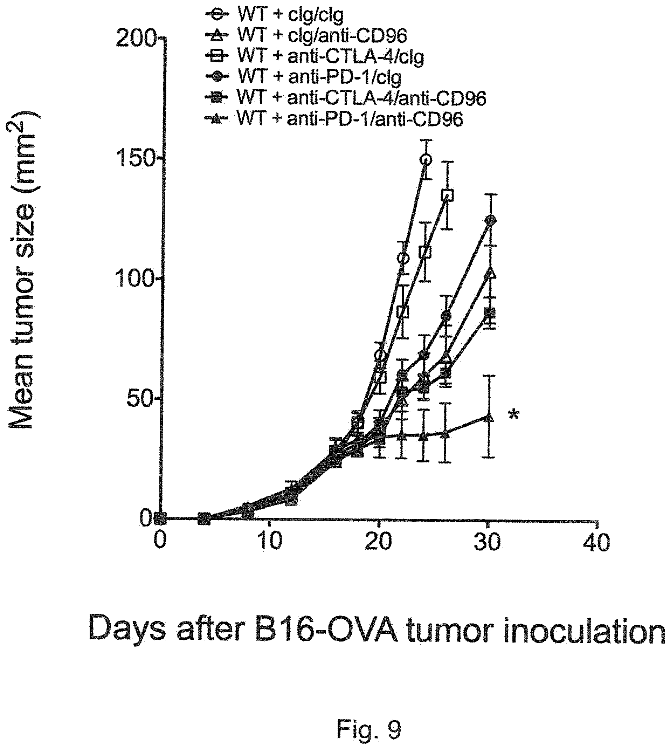

Figure 8: Early anti-CD96 mAb enhances anti-tumor responses generated by anti-PD-1 and anti-CTLA-4 mAbs. C57BL/6 wild-type (WT) mice were injected subcutaneously with B16-OVA melanoma cells (10⁵ cells) and treated on day 1, 5, and 9 with intraperitoneal injections of anti-CD96 mAb (3.3, 250 µg i.p), anti-PD-1 mAb (RMP1-14, 250 µg i.p.), anti-CTLA-4 (UC10-4F10, 250 µg i.p.), anti-CD96/anti-PD-1 mAbs (250 µg i.p each), anti-CD96/anti-CTLA-4 mAbs (250 µg i.p each) or control Ig (cIg) (2A3, 250 µg i.p). Means ± SEM of 5 mice per group (mm²) are shown (*: p<0.05 compared with anti-CD96 alone, by Mann-Whitney test). Figure 9: Late anti-CD96 mAb enhances anti-tumor responses generated by anti-PD-1 mAb. C57BL/6 wild-type (WT) mice were injected subcutaneously with B16-OVA melanoma cells (10⁵ cells) and treated on day 16, 20, and 24 with intraperitoneal injections of anti-CD96 mAb (3.3, 250 µg i.p), anti-PD-1 mAb (RMP1-14, 250 µg i.p.), anti-CTLA-4 (UC10-4F10, 250 µg i.p.), anti-CD96/anti-PD-1 mAbs (250 µg i.p each), anti-CD96/anti-CTLA-4 mAbs (250 µg i.p each) or control Ig (cIg) (2A3, 250 µg i.p). Means ± SEM of 5 mice per group (mm²) are shown (*: p<0.05 compared with anti-CD96 alone by Mann-Whitney test).

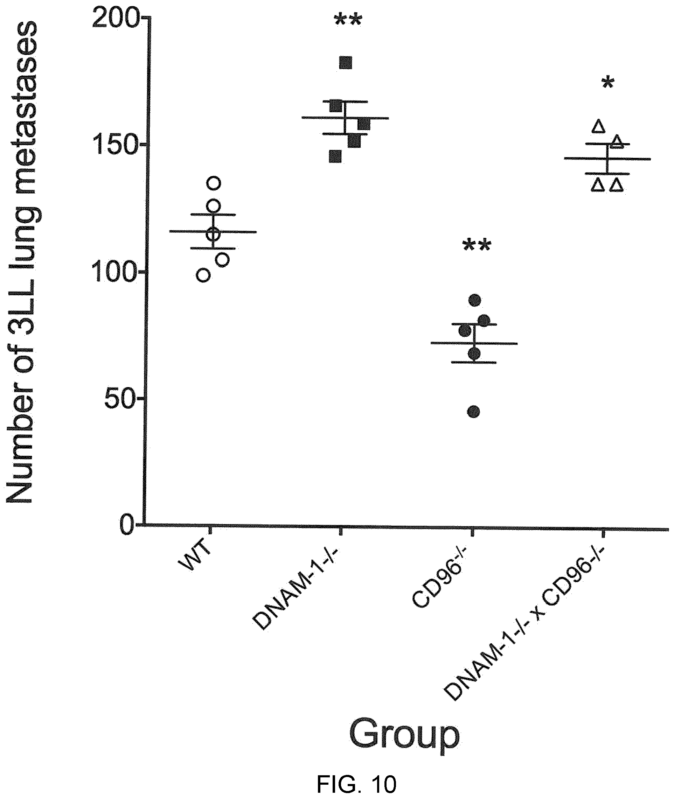

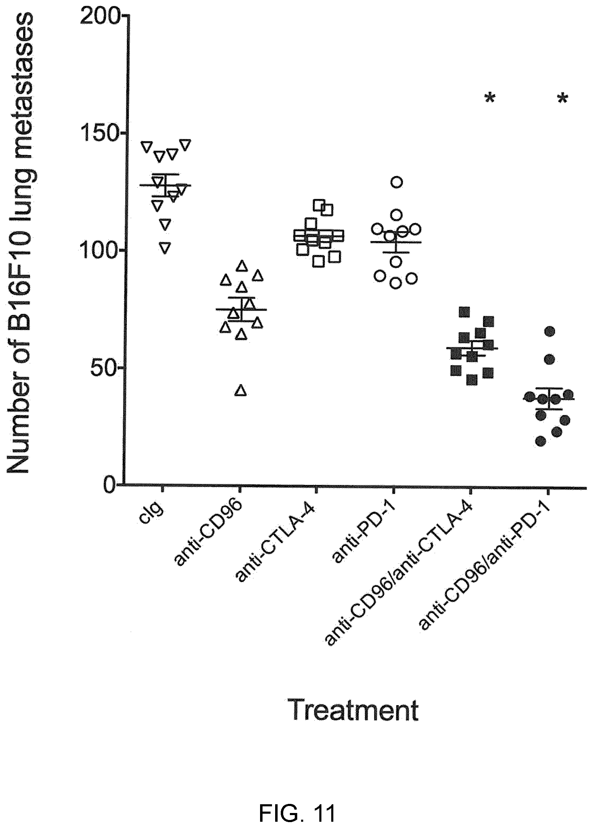

Figure 10: Host CD96 promotes 3LL lung metastasis. C57BL/6 wild type (WT), DNAM-1^{-/-}, CD96^{-/-}, and DNAM-1^{-/-}CD96^{-/-} mice were injected intravenously with 3LL lung carcinoma cells (10⁵ cells) and metastatic burden was quantified in the lungs after 14 days by counting colonies on the lung surface. Means ± SEM of 5 mice per group are shown (*: p<0.05 compared with WT by Mann-Whitney test). Figure 11: Anti-CD96 suppresses B16F10 lung metastasis, alone and in combination with T cell checkpoint blockade. C57BL/6 wild type (WT) mice were injected intravenous with B16F10 melanoma cells (10⁵ cells). On day 0 and 3 after tumor inoculation, mice were treated with intraperitoneal injections of anti-CD96 mAb (3.3, 250 µg i.p), anti-PD-1 mAb (RMP1-14, 250 µg i.p.), anti-CTLA-4 (UC10-4F10, 250 µg i.p.), anti-CD96/anti-PD-1 mAbs (250 µg i.p each), anti-CD96/anti-CTLA-4 mAbs (250 µg i.p each) or control Ig (cIg) (2A3, 250 µg i.p). Metastatic burden was quantified in the lungs after 14 days by counting colonies on the lung surface. Means ± SEM of 5 mice per group are shown (*: p<0.05 compared with anti-CD96 alone by Mann-Whitney test).

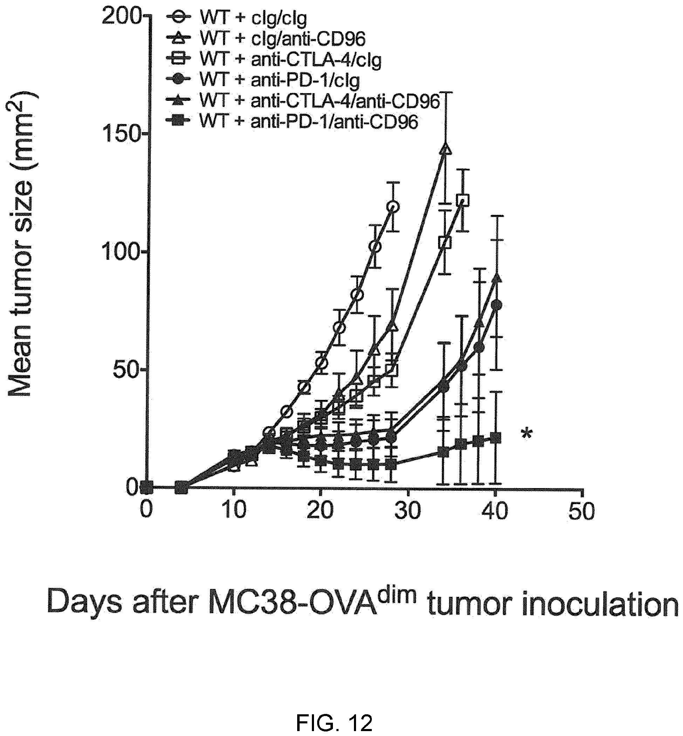

Figure 12: Late anti-CD96 mAb enhances anti-tumor responses generated by anti-PD-1 mAb. C57BL/6 wild-type (WT) mice were injected subcutaneously with MC38-OVA^{dim} colon adenocarcinoma cells (10⁶ cells) and treated on day 14, 18, 22, and 26 with intraperitoneal injections of anti-CD96 mAb (3.3, 250 µg i.p), anti-PD-1 mAb (RMP1-14, 250 µg i.p.), anti-CTLA-4 (UC10-4F10, 250 µg i.p.), anti-CD96/anti-PD-1 mAbs (250 µg i.p each), anti-CD96/anti-CTLA-4 mAbs (250 µg i.p each) or control Ig (cIg) (2A3, 250 µg i.p). Means ± SEM of 5 mice per group (mm²) are shown (*: p<0.05 compared with anti-CD96 alone by Mann-Whitney test).

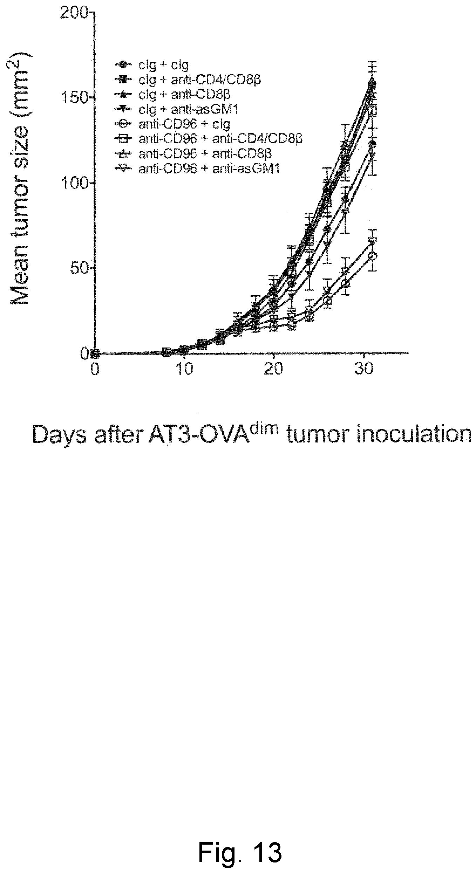

Figure13. Mechanism of the anti-tumor effect of anti-CD96 alone against AT3-OVA^{dim} mammary carcinoma. C57BL/6 wild-type (WT) mice were injected s.c. with AT3-OVA^{dim} mammary carcinoma (1 × 10⁶ cells). On day 16, 20, and 24 after tumor inoculation mice were treated with i.p. injections of cIg (250 µg i.p.) or anti-CD96 mAb (250 µg i.p.). Some groups of mice were treated on days 14, 16, and 23 after tumor inoculation with cIg, anti-CD4/anti-CD8β (100 µg i.p.) or anti-asGM1 (100 µg i.p.).

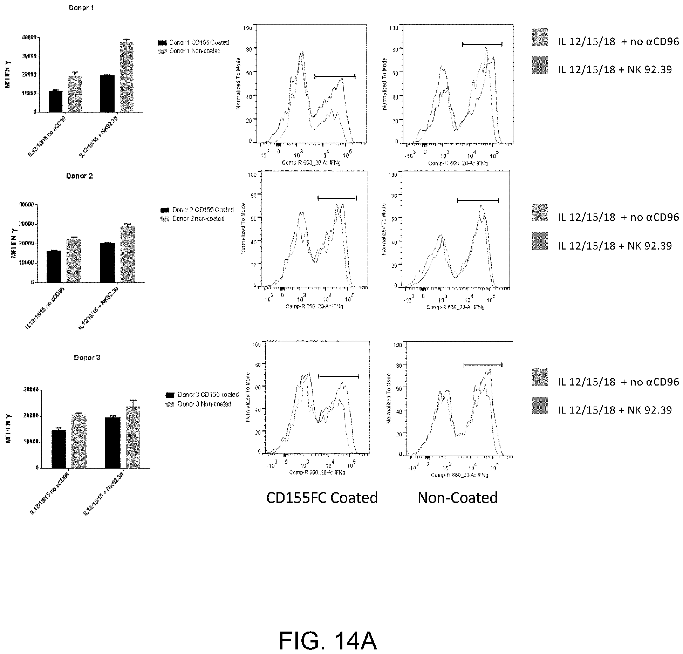

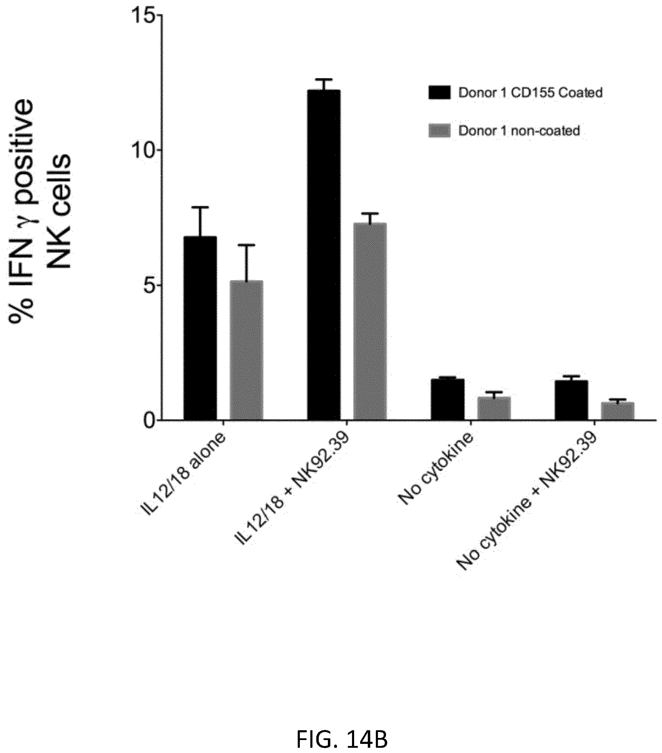

Figure 14. In Vitro NK cell activation. To analyse the production of IFNγ from human NK cells, 96 well U bottom plates were coated with recombinant human CD155-Fc chimera overnight at 4°C (0.25µg/well). After three washes, 2.5 × 10⁴ human NK cells, freshly isolated from buffy coats and FACS sorted were plated in complete RPMI supplemented with human IL-12 (10 ng/ml), IL-15 (100 ng/ml) and IL-18 (100 ng/ml) in the presence or absence of human anti-CD96 antibody (clone NK92.39, 50 µg/ml). Cultures were also set up in wells not containing CD155-Fc coating. After 24 hours incubation, cells were harvested and analysed for IFNγ production by Flow Cytometry. All conditions were run in triplicate and error bars represent the ±SEM. (A) Flow cytometry results using IL-12, 18 and 15; and (B) result from a different donor (not donor 1 shown in 17A) showing the proportion of IFNγ positive NK cells.

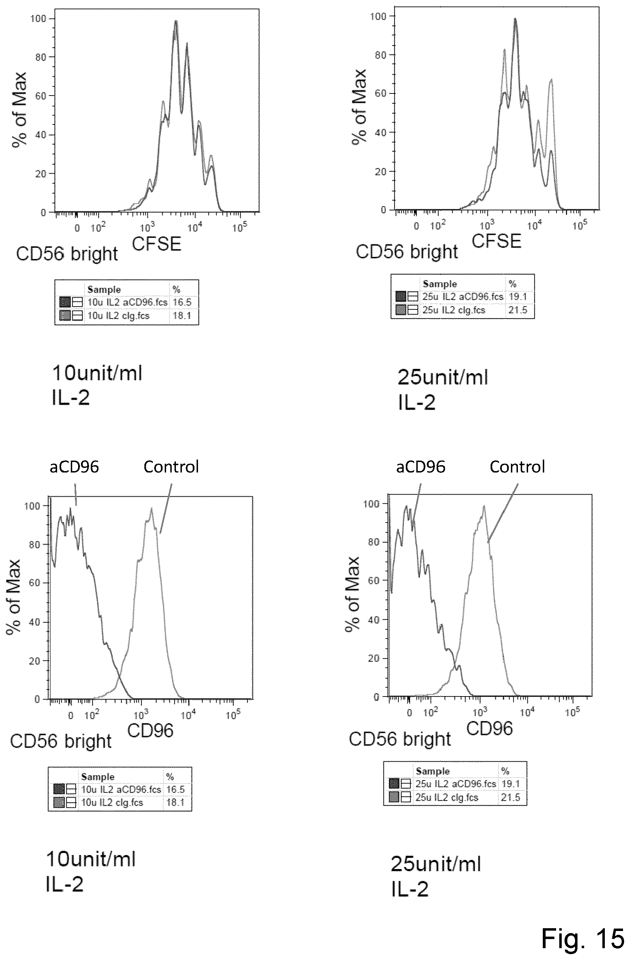

Figure 15. Binding of human CD96 mAb (NK92.39) to human NK cells reduced the levels of CD96 present on the NK cell surface. Total NK cells were purified from peripheral blood mononuclear cells (PBMCs) by negative selection using human NK cell isolation kit (Miltenyi Biotec.). Isolated NK cells were then labeled with carboxyfluorescein diacetate succinmidyl ester (CFSE; Biolegend) to measurecellular proliferation. CFSE-labeled NK cells were plated in 96 well U-bottom plate at 5 × 10⁴ cells/well and stimulated with recombinant IL-2 at indicated concentrations (10 units/ml and 25 units/ml), in the presence of control IgG or anti-CD96 mAb (clone NK92-39) at 30 µg/ml. NK cells were assessed for changes in proliferation (A) or the presence/absence of surface CD96; and (B) at day 3 and 6 using BD FACS Canto II (BD Biosciences) and analysis was carried out using FlowJo (Tree Star).

### DETAILED DESCRIPTION

The present invention is at least partly predicated on the unexpected discovery that CD96 is highly expressed by resting NK cells and T cell subsets and competes with DNAM-1 for the binding of CD155 on resting NK cells. Using CD96^{-/-} mice, it is demonstrated that CD96 dampens or suppresses NK cell production of IFN-γ *in vitro* and *in vivo,* through competition with DNAM-1 for CD155 binding and also through a direct inhibition. Furthermore, CD96^{-/-} mice were shown to be more resistant to 3'-methylcholanthrene (MCA)-induced tumor formation as an indicator of carcinogenesis, or B16F10 (melanoma), RM-1 (prostate cancer), 3LL (lung cancer) experimental metastasis. In human NK cells, administration of anti-CD96 antibody appears to remove cell surface CD96 and/or cause a down-regulation of CD96 cell surface expression. Based on these observations, it is proposed that CD96 normally acts as a negative regulator of T and NK cell anti-tumor functions, particularly although not exclusively through suppression of IFN-γ production and/or secretion. Accordingly, the invention provides methods of relieving or reducing the negative immunoregulatory function of CD96 to thereby promote or restore immune surveillance, particularly by T cells and NK cells, to thereby treat or prevent cancer, cancer cell metastasis and/or viral infections.

An aspect of the invention therefore provides a method of reducing or relieving immune inhibition in a mammal, said method including the step of at least partly inhibiting or reducing CD96 activity in one or more cells of the mammal to thereby relieve immune inhibition and/or enhance or restore immune surveillance in the mammal.

By *"relieving immune inhibition"* in the context of CD96 is meant at least partly eliminating, removing or overcoming a normal activity or function of CD96 in suppressing or inhibiting one or more immune functions of cells that normally express CD96. Typically, the one or more cells that normally express CD96 are T cells, inclusive of CD4⁺ and CD8⁺ T cells, γδT cells, NKT cells, and natural killer (NK) cells. In some embodiments, relieving immune inhibition may include or relate to abrogating peripheral tolerance to foreign pathogens, host cells displaying foreign pathogens (*e.g* displaying foreign pathogen-derived peptides in the context of self-MHC) and/or cancerous cells or tissues of the host.

By *"enhance or restore immune surveillance"* is meant at least partly improving or promoting the ability of one or more elements of the immune system to monitor, detect and/or respond to foreign pathogens, host cells displaying foreign pathogens (*e.g* displaying foreign pathogen-derived peptides in the context of self-MHC) and/or cancerous cells or tissues of the host. Suitably, the elements of the immune system are one or more cells that normally express CD96, such as T cells, inclusive of CD4⁺ and CD8⁺ T cells γδT cells, NKT cells and natural killer (NK) cells.

At least partly inhibiting or reducing CD96 activity in one or more cells of the mammal may be performed, facilitated or achieved by administration of a *"CD96-inhibitory agent"* to the mammal. A CD96-inhibitory agent may be any molecule that possesses or displays an ability to at least partly inhibit or reduce a biological activity of CD96. Biological activities of CD96 include one or more of binding CD155, removing cell surface CD96, reducing cell surface expression, eliciting intracellular signaling and/or stimulating or inducing expression and/or secretion of cytokines or chemokines. Preferably, the cytokines or chemokines include any pro-inflammatory cytokine or chemokine inclusive of MIP-1α, MIP-1β, RANTES, TNF-α and IFN-γ, although without limitation thereto. Preferably, the cytokine is IFN-γ.

As disclosed herein, CD96 is a transmembrane protein which in the human, exists in two isoforms. Isoform 1 has been detected in acute myeloid leukaemia and includes additional amino acids compared with isoform 2. Isoform 2 is the more common form in humans and the predicted domain architecture of isoform 2 has three (3) external immunoglobulin-like domains (domains 1, 2 and 3) as listed in Table 1. The amino acid sequence of human CD96 isoform 1 is set forth in SEQ ID NO:2. The nucleotide sequence encoding isoform 2 is set forth in SEQ ID NO:1 Murine CD96 exists as a single isoform, the amino acid sequence of which is set forth in SEQ ID NO:4. The nucleotide sequence encoding murine CD96 is set forth in SEQ ID NO:3 The external immunoglobulin-like domains (domains 1, 2 and 3) of murine CD96 are also listed in Table 1.

In a preferred form, the CD96-inhibitory agent binds or interacts with an amino acid sequence of one or a plurality of external immunoglobulin-like domains of CD96. For example, the CD96-inhibitory agent may bind or interact with an amino acid sequence of: domain 1; domain 2; domain 3; domain 1 and domain 2; domain 1 and domain 3: domain 2 and domain 3; or domain 1, domain 2 and domain 3.

In one embodiment, the CD96-inhibitory agent binds or interacts with one or a plurality of external immunoglobulin-like domains of human CD96 isoform 2.

It will also be appreciated that the CD96-inhibitory agent may bind or interact with other CD96 domains or amino acid sequences in addition to one or a plurality of the external or extracellular immunoglobulin-like domains.

In one embodiment, the CD96-inhibitory agent inhibits, blocks or antagonizes a binding interaction between CD96 and CD155. By way of example only, the CD96-inhibitory agent may bind to an extracellular domain of CD96, or a portion thereof, that is capable of interacting with CD155 (*e.g.* binding CD155 or being bound by CD155) to thereby at least partly inhibit or block CD96 binding to CD155.

In another embodiment, the CD96-inhibitory agent is a molecule that possesses or displays an ability to inhibit or reduce CD96 signaling activity. Inhibition or reduction of CD96 signaling activity may be through inhibiting, blocking or antagonizing a binding interaction with CD155 or may be through blocking CD96-initiated signaling that would normally occur in response to CD155 binding. By way of example, CD96 comprises an immunoreceptor tyrosine-based inhibitory motif (ITIM). ITIMs are structurally defined as 6-amino acid sequences comprising a tyrosine (Y) residue with partly conserved N-terminal (Y-2) and C-terminal (Y+3) residues. A general but non-limiting motif is (S/I/V/LXYXXI/V/L), wherein X is any amino acid. For example, isoform 1 of CD96 comprises the ITIM sequence IKYTCI wherein Y is residue 566.

It has been proposed that when co-aggregated with activating receptors, ITIMs are phosphorylated by Src-family tyrosine kinases, which enables them to recruit Src homology 2 domain-containing phosphatases (PTPases) that antagonize activation signals. Accordingly, in one embodiment the CD96-inhibitory agent possesses or displays an ability to inhibit or reduce CD96 signaling activity mediated by the CD96 ITIM. Preferably, inhibition or reduction of CD96 signaling activity mediated by the CD96 ITIM enables increased or enhanced cytokine (*e.g* IFN-γ) expression, production and/or secretion by a cell expressing CD96.

In another embodiment, the CD96-inhibitory agent is a molecule that removes cell surface CD96 and/or reduces or down-regulates cell surface expression of CD96.

The CD96-inhibitory agent may be a protein (inclusive of peptides, antibodies and antibody fragments), a nucleic acid (inclusive of inhibitory RNA molecules such as ribozymes, RNAi, miRNA and siRNA, although without limitation thereto), a lipid, a carbohydrate, a small organic molecule or any combination of these (*e.g* a glycoprotein, a lipoprotein, a peptide-nucleic acid *etc*)*.*

In one particular embodiment, the CD96-inhibitory agent is an antibody or antibody fragment that binds CD96.

In a preferred form, the antibody or antibody fragment binds or interacts with an amino acid sequence of one or a plurality of external or extracellular immunoglobulin-like domains of CD96. For example, the antibody or antibody fragment may bind or interact with an amino acid sequence of: domain 1; domain 2; domain 3; domain 1 and domain 2; domain 1 and domain 3: domain 2 and domain 3; or domain 1, domain 2 and domain 3.

In one embodiment, the antibody binds or interacts with one or a plurality of external immunoglobulin-like domains of human CD96 isoform 2.

In one form the antibody binds CD96 and at least partly blocks or inhibits CD96 binding to CD155.

Antibodies may be polyclonal or monoclonal, native or recombinant. Antibody fragments include Fab and Fab'2 fragments, diabodies and single chain antibody fragments (*e.g.* scVs), although without limitation thereto. Antibodies and antibody fragments may be modified so as to be administrable to one species having being produced in, or originating from, another species without eliciting a deleterious immune response to the "foreign" antibody. In the context of humans, this is "humanization" of the antibody produced in, or originating from, another species. Such methods are well known in the art and generally involve recombinant "grafting" of non-human antibody complementarity determining regions (CDRs) onto a human antibody scaffold or backbone.

Suitably, the step of inhibiting or reducing CD96 activity in the mammal does not include killing CD96-expressing cells in the mammal. In this context, "killing" may refer to any antibody-mediated cytotoxic mechanism such as complement-mediated cytolysis and antibody-mediated cell-mediated cytotoxicity (ADCC), the latter typically mediated by natural killer (NK) cells, macrophages, neutrophils and eosinophils. In this regard, it may be advantageous to use antibody fragments lacking an Fc portion or having a mutated Fc portion.

The step of inhibiting or reducing CD96 activity in the mammal may be achieved or facilitated by administering a CD96-inhibitory agent to the mammal.

By *"administering"* is meant the introduction of the CD96-inhibitory agent into the mammal by a particular route. Suitably, a therapeutically effective amount of the CD96-inhibitory agent is administered to the mammal.

The term *"therapeutically effective amount"* describes a quantity of a specified agent sufficient to achieve a desired effect in a mammal being treated with that agent.

Generally, the method of the invention may be useful in reducing or relieving CD96-mediated immune inhibition, suppression or peripheral tolerance. Suitably, the method facilitates treatment or prevention of one or more diseases or conditions that are responsive to at least partly blocking CD96-mediated immune inhibition, suppression or peripheral tolerance.

As used herein, *"treating"* or *"treat"* or *"treatment"* refers to a therapeutic intervention that at least party eliminates or ameliorates one or more existing or previously identified symptoms of a disease or condition that is responsive to at least partly blocking CD96-mediated immune inhibition, suppression or peripheral tolerance.

As used herein, *"preventing"* or *"prevent"* or *"prevention"* refers to a prophylactic treatment initiated prior to the onset of a symptom of a disease or condition that is responsive to at least partly blocking CD96-mediated immune inhibition, suppression or peripheral tolerance, so as to at least partly or temporarily prevent the occurrence of the symptom.

Typically, the disease or condition that is responsive to at least partly blocking CD96-mediated immune inhibition, suppression or peripheral tolerance is any disease or condition where enhanced or restored immune surveillance can benefit a subject suffering from the disease or condition. Such diseases and conditions may include those where persistence of the disease or condition can be controlled or suppressed by cell-mediated immunity. Non-limiting examples include cancers and viral infections. Particular viral infections contemplated by the invention include persistent viral infections such as caused by human immunodeficiency virus (HIV), Epstein Barr Virus (EBV), Herpes Simplex Virus (HSV inclusive of HSV1 and HSV2), Human Papillomavirus (HPV), Varicella zoster virus (VSV) and Cytomegalovirus (CMV), although without limitation thereto.

In a preferred embodiment, the method reduces or relieves immune inhibition in a mammal sufficient to treat or prevent cancer or cancer metastasis in the mammal. Suitably, the cancer may be any that is responsive to at least partly blocking CD96-mediated immune inhibition, suppression or peripheral tolerance. Cancers may be in the form of solid tumors, sarcomas, lymphomas, myelomas, carcinomas, melanomas, cytomas and meningiomas, although without limitation thereto. Non-limiting examples of cancers include cancers of the adrenal gland, bladder, bone, bone marrow, brain, breast, cervix, gall bladder, ganglia, gastrointestinal tract, heart, kidney, liver, lung, muscle, ovary, pancreas, pituitary, parathyroid, prostate, salivary glands, skin, spleen, testis, thyroid, and uterus. Particular non-limiting examples of cancers include colon cancer, lung cancer and prostate cancer. In some embodiments, the cancer is a metastatic cancer, which is capable of migrating to another site, tissue or organ in the body and forming a tumor at that site, tissue or organ. This may occur repeatedly over time. A particularly aggressive metastatic cancer contemplated by the invention is metastatic melanoma.

It will also be appreciated that the method of treatment or prevention of cancer may further include co-administration of one or more other therapeutic agents that facilitate cancer treatment or prevention. By way of example only, these include: chemotherapeutic agents such as paclitaxel, doxorubicin, methotrexate and cisplatin, although without limitation thereto; and/or biotherapeutic agents such as anti-PD-1 antibodies (*e.g.* Nivolumab) and anti-CTLA4 antibodies (*e.g* Ipilimumab), although without limitation thereto. Also contemplated are bi-specific antibodies that bind both CD96 and one or more other molecules including but not limited to PD-1 and CTLA4.

The one or more other agents that facilitate cancer treatment or prevention may be administered in combination with the CD96-inhibitory agent or be administered separately, as is well understood in the art.

In some embodiments, the CD96-inhibitory agent may be formulated alone or together with the one or more other agents is in the form of a pharmaceutical composition.

Suitable dosages of CD96-inhibitory agents, alone or together with other therapeutic agents, for mammalian administration, including human administration, may be readily determined by persons skilled in the art.

Suitably, the pharmaceutical composition comprises an appropriate pharmaceutically-acceptable carrier, diluent or excipient.

Preferably, the pharmaceutically-acceptable carrier, diluent or excipient is suitable for administration to mammals, and more preferably, to humans.

By *"pharmaceutically-acceptable carrier, diluent or excipient"* is meant a solid or liquid filler, diluent or encapsulating substance that may be safely used in systemic administration. Depending upon the particular route of administration, a variety of carriers, diluents and excipients well known in the art may be used. These carriers may be selected from a group including sugars, starches, cellulose and its derivatives, malt, gelatine, talc, calcium sulfate, vegetable oils, synthetic oils, polyols, alginic acid, phosphate buffered solutions, emulsifiers, isotonic saline and salts such as mineral acid salts including hydrochlorides, bromides and sulfates, organic acids such as acetates, propionates and malonates, and pyrogen-free water.

A useful reference describing pharmaceutically acceptable carriers, diluents and excipients is Remington's Pharmaceutical Sciences (Mack Publishing Co. NJ USA, 1991).

Any safe route of administration may be employed for providing a subject with compositions comprising the CD96-inhibitory agent. For example, oral, rectal, parenteral, sublingual, buccal, intravenous, intra-articular, intra-muscular, intradermal, subcutaneous, inhalational, intraocular, intraperitoneal, intracerebroventricular, transdermal, and the like may be employed.

A further aspect of the invention provides a method of screening, designing, engineering or otherwise producing a CD96-inhibitory agent, said method including the step of determining whether a candidate molecule is capable of at least partly inhibiting or reducing CD96 activity to thereby relieve immune inhibition and/or enhance or restore immune surveillance in a mammal.

The invention also provides a CD96-inhibitory agent screened, designed, engineered or otherwise produced according to the aforementioned aspect.

The candidate molecule may be a protein (inclusive of peptides, antibodies and antibody fragments), a nucleic acid (inclusive of inhibitory RNA molecules such as ribozymes, RNAi, miRNA and siRNA, although without limitation thereto), a lipid, a carbohydrate, a small organic molecule or any combination of these (*e.g* a glycoprotein, a lipoprotein, a peptide-nucleic acid *etc*)*.*

In some embodiments, the candidate modulator may be rationally designed or engineered *de novo* based on desired or predicted structural characteristics or features that indicate the candidate modulator could block or inhibit one or more biological activities of CD96, such as CD155 binding, intracellular signaling and/or IFN-γ production and/or secretion. In other embodiments, the candidate modulator may be identified by screening a library of molecules without initial selection based on desired or predicted structural characteristics or features that indicate the candidate modulator could block or inhibit one or more biological activities of CD96. Such libraries may comprise randomly generated or directed libraries of proteins, peptides, nucleic acids, recombinant antibodies or antibody fragments (*e.g.* phage display libraries), carbohydrates and/or lipids, libraries of naturally-occurring molecules and/or combinatorial libraries of synthetic organic molecules.

Non-limiting examples of techniques applicable to the design and/or screening of candidate modulators may employ X-ray crystallography, NMR spectroscopy, computer assisted screening of structural databases, computer-assisted modelling or biochemical or biophysical techniques which detect molecular binding interactions, as are well known in the art.

Biophysical and biochemical techniques which identify molecular interactions include competitive radioligand binding assays, co-immunoprecipitation, fluorescence-based assays including fluorescence resonance energy transfer (FRET) binding assays, electrophysiology, analytical ultracentrifugation, label transfer, chemical cross-linking, mass spectroscopy, microcalorimetry, surface plasmon resonance and optical biosensor-based methods, such as provided in Chapter 20 of CURRENT PROTOCOLS IN PROTEIN SCIENCE Eds. Coligan et al., (John Wiley & Sons, 1997) Biochemical techniques such as two-hybrid and phage display screening methods are provided in Chapter 19 of CURRENT PROTOCOLS IN PROTEIN SCIENCE Eds. Coligan et al., (John Wiley & Sons, 1997).

Accordingly, an initial step of the method may include identifying a plurality of candidate molecules that are selected according to broad structural and/or functional attributes, such as an ability to bind CD96.

The method may include a further step that measures or detects a change in one or more biological activities of CD96 in response to the candidate molecule(s). These may include CD155 binding, the presence or absence of cell surface CD96, cell surface expression, intracellular signaling, cytokine and/or chemokine production or secretion and/or protection from tumor challenge in an *in vivo* model.

Inhibition of CD155 binding to CD96 by a candidate molecule may be determined by any of several techniques known in the art including competitive radioligand binding assays, surface plasmon resonance (*e.g.* BIACore^{™} analysis), co-immunoprecipitation and fluorescence-based analysis of the ability of a candidate inhibitor to block CD155 binding to CD96 (such as by flow cytometry where CD155 is labeled with a fluorophore). Non-limiting examples of fluorophores include fluorescein isothiocyanate (FITC), allophycocyanin (APC), fluoroscein derivatives such as FAM and ROX, Texas Red, tetramethylrhodamine isothiocyanate (TRITL), R-Phycoerythrin (RPE), Alexa and Bodipy fluorophores, although without limitation thereto.

Alternatively, this fluorescence-based analysis could include FRET analysis (*e.g.* one protein coupled to a donor fluorophore, the other coupled to an acceptor fluorophore), although without limitation thereto.

In some embodiments, intracellular signaling may be measured directly at the level of CD96, such as by measuring recruitment of SH2 domain-containing PTPases by CD96 expressed by NK cells, or T cell subsets. A candidate molecule of the invention suitably prevents or reduces recruitment of SH2 domain-containing PTPases by CD96 in the presence of CD155. According to this embodiment, the candidate molecule may at least partly inhibit or prevent binding between CD96 and CD155, thereby at least partly inhibiting or preventing intracellular signaling by CD96, and/or at least partly inhibit or prevent intracellular signaling by CD96 despite CD155 binding.

In other embodiments, an effect of a candidate molecule on CD96 may be determined by measuring expression, production and/or secretion of one or more cytokines or chemokines by cells expressing CD96. Generally, changes in cytokine or chemokine expression production and/or secretion may be measured by RT-PCR of cytokine mRNA, measurement of cytokine or chemokine protein located intracellularly (*e.g* by immunocytochemistry using cytokine- or chemokine-specific antibodies) and/or measurement of secreted cytokines or chemokines such as by flow cytometric Cytokine Bead Array (such as commercially available from BD Biosciences), by ELISA using cytokine- or chemokine-specific antibodies and by bioassays that use cytokine- or chemokine-responsive cell lines to measure cytokines and/or chemokines secreted into cell supernatants. The cytokine may be any pro-inflammatory cytokine or chemokine inclusive of MIP-1α, MIP-1β, RANTES, TNF-α and IFN-γ, although without limitation thereto. Preferably, the cytokine is IFN-γ.

The effect of a candidate CD96 inhibitory agent may be upon CD96 expression, inclusive of cell surface expression and CD96 gene expression. It will be appreciated that in certain cells, such as NK cells although without limitation thereto, CD96 inhibitory agents may cause or facilitate down-regulation of CD96 expression. In some embodiments, thus may include down-regulation of CD96 expression at the cell-surface. This may occur as a result of enhanced internalization or endocytosis of cell surface CD96 and/or by a down-regulation or suppression of CD96 gene expression. In particular embodiments, CD96 cell surface expression may be detected or measured by flow cytometry, immunprecipitation, immunocytochemistry or immunohistochemistry, typically by way of an antibody or antibody fragment which binds CD96, as hereinbefore described. In particular embodiments, CD96 gene expression may be measured by nucleic acid sequence amplication (e.g. PCR) or nucleic acid hybridization techniques such as Northern blotting.

Preferably, the CD96-inhibitory effect of a candidate molecule may be determined using an *in vivo* tumor challenge model. For example, a mouse model using CD96-expressing mice may be used to determine the ability of a candidate molecule to inhibit or prevent tumor formation and/or growth in response to an administered carcinogenic agent such as methycholanthrene (MCA). In another example, a mouse model using CD96-expressing mice may be used to determine the ability of a candidate molecule to inhibit or prevent tumor formation and/or growth in response to administration of tumor cells such as melanomas, colon adenocarcinomas, prostate carcinomas and mammary carcinomas, although without limitation thereto. Other mouse models may utilize mice that are predisposed to spontaneously forming tumors including but not limited to MMTV-polyoma, MT mammary cancer, DMBA/TPA induced skin cancer, p53 loss lymphomalsarcoma and TRAMP Tg prostate cancer.

It will be understood that the method of this aspect may be performed iteratively, whereby multiple rounds of screening, design, and biological testing are performed. This may include where a candidate molecule is structurally modified before each round, thereby enabling "fine-tuning" of the candidate molecule.

It will also be appreciated that the method may be performed in a "high throughput", "automated" or "semi-automated" manner, particularly during early stages of candidate molecule identification and selection.

In a preferred embodiment, the candidate molecule is an antibody or antibody fragment. As hereinbefore described, antibodies may be polyclonal or monoclonal, native or recombinant. Antibody fragments include Fab and Fab'2 fragments, diabodies and single chain antibody fragments (*e.g.* scVs), although without limitation thereto. Well-known protocols applicable to antibody production, selection, purification and use may be found, for example, in Chapter 2 of Coligan et al., CURRENT PROTOCOLS IN IMMUNOLOGY (John Wiley & Sons NY, 1991-1994) and Harlow, E. & Lane, D. Antibodies: A Laboratory Manual, Cold Spring Harbor, Cold Spring Harbor Laboratory, 1988, which are both herein incorporated by reference.

Polyclonal antibodies may be prepared for example by injecting CD96 or a fragment (*e.g* a peptide) thereof into a production species, which may include mice or rabbits, to obtain polyclonal antisera. Methods of producing polyclonal antibodies are well known to those skilled in the art. Exemplary protocols that may be used are described for example in Coligan et al., CURRENT PROTOCOLS IN IMMUNOLOGY, *supra,* and in Harlow & Lane, 1988, *supra.*

Monoclonal antibodies may be produced using the standard method as for example, described in an article by Köhler & Milstein, 1975, Nature 256, 495, which is herein incorporated by reference, or by more recent modifications thereof as for example, described in Coligan et al., CURRENT PROTOCOLS IN IMMUNOLOGY, *supra* by immortalizing spleen or other antibody producing cells derived from a production species which has been inoculated with one or more of the isolated proteins, fragments, variants or derivatives of the invention. Suitably, the antibody or antibody fragment is suitable for administration to a human. In this context, as hereinbefore described the antibody or antibody fragment may be a "humanized" form of an antibody or antibody fragment produced in, or originating from, another species. Such methods are well known in the art and generally involve recombinant "grafting" of non-human antibody complementarity determining regions (CDRs) onto a human antibody scaffold or backbone.

In a preferred embodiment, the antibody or antibody fragment does not kill CD96-expressing cells upon administration to a human. In this context, "killing" may refer to any antibody-mediated cytotoxic mechanism such as complement-mediated cytolysis and antibody-mediated cell-mediated cytotoxicity (ADCC), the latter typically mediated by natural killer (NK) cells, macrophages, neutrophils and eosinophils. In this regard, it may be advantageous to use antibody fragments lacking an Fc portion or having a human Fc portion (*e.g* a humanized antibody).

A CD96-inhibitory agent screened, designed, engineered or otherwise produced according to the aforementioned aspect may be used according to the method of the first aspect (*e.g* as an anti-cancer agent and/or an anti-viral agent), preferably in the form of a pharmaceutical composition as hereinbefore described.

So that the invention may be readily understood and put into practical effect, reference is made to the following non-limiting examples.

### EXAMPLES

### Example 1

### CD96 binding to CD155 and the effects of CD96 inhibition and knockout in mouse tumor models

### Materials and Methods

### Mice

Wild Type C57BL/6 mice were purchased from the Walter and Eliza Hall Institute for Medical Research or ARC Animal Resource Centre. C57BL/6 CD96^{-/-}mice were generated by Dr. Marco Colonna and Dr. Susan Gilfillan at the Washington University School of Medicine (St Louis, MO, USA) as follows. A targeting construct designed to replace exons 1 and 2 of CD96, including the start site, with a MC1-neor gene flanked by loxP sites was electroporated into E14.1 (129P2/OlaHsd) embryonic stem cells (Fig. S1). Chimeras transmitting the targeted allele were obtained from two clones following injection into C57BL/6 blastocysts. Mice carrying the targeted allele were bred to C57BL/6 mice expressing *a Cre* transgene under the CMV promoter to delete the MC1-neor gene (Schwenk et al., 1995). The CD96 deletion was backcrossed onto a C57BL/6 background, facilitated by a genome-wide screening of polymorphic microsatellite markers at 10-centiMorgan intervals at each generation. CD96^{+/-} >99% C57BL/6 mice were intercrossed to generate the CD96^{-/-} mice. DNAM-I^{-/-} mice have already been described. DNAM-1^{-/-} CD96^{-/-} were generated by intercrossing CD96^{-/-} with DNAM-1^{-/-} mice. Mice were bred and used between the ages of 6-14 weeks. All experiments were approved by animal ethics committee.

### Cell Culture

B16F10, RM-1, 3LL, AT3, MC38 and YAC-1 cell lines were grown in complete RPMI Medium (Gibco, Invitrogen,) i.e supplemented with 10% FCS (Thermo Scientific), L-Glutamine (Gibco), Non-Essential Amino Acids, Sodium Pyruvate, HEPES (Gibco) and Penicillin/Streptomycin (Gibco), at 37 ⁰ C in 5% CO₂. For cytotoxicity assays and IL-12/IL-18 titration experiments, primary NK cells were harvested from the spleen, sorted using a mouse NK cell isolation kit (Miltenyi Biotec) and AutoMACS (Miltenyi Biotec), and subsequently cultured for 5 days in RPMI Medium supplemented with 10% FCS, L-Glutamine, Penicillin/Streptomycin, Non-Essential Amino Acids (Gibco), Sodium Pyruvate (Gibco), HEPES (Gibco), β-2-mercaptoethanol (Calbiochem), and 1000 IU/ml recombinant human IL-2 (Chiron Corporation). All cells were incubated at 37°C in 5% CO₂.

### In vivo LPS challenges

LPS (from *E. Coli* 0127:B8, Sigma) suspended in PBS was injected intraperitoneally into mice at the described doses. For survival curves, mice were checked hourly for symptoms of sepsis. Serum from these mice was taken at various time points by retro-orbital or cardiac bleeding for cytokine analysis. Spleens were also taken from mice at various time points to analyse receptor and ligand expression, and intracellular IFN-γ expression from NK cells.

### In vivo tumor challenges

Mouse B16F10 or B16-OVA melanomas, RM-1 prostate carcinoma, 3LL lung carcinoma, MC38-OVA^{dim} colon adenocarcinoma or AT3-OVA^{dim} mammary carcinoma, were injected into WT, DNAM-1^{-/-}, CD96^{-/-}, or DNAM-1^{-/-}CD96^{-/-} mice subcutaneously or intravenously at the indicated doses and monitored for solid tumor growth or metastasis, respectively. Treatments were administered as indicated in the Figure legends. To monitor solid tumor growth, the area of the ensuing tumor was calculated by taking the length and width of palpable tumors by calipers and plotted against time. To monitor metastasis formation, 14 days after cells were injected, lungs were harvested, placed in Bouin's fixative, and metastases were counted using a dissection microscope.

### MCA -induced fibrosarcoma

WT, DNAM-1^{-/-}, CD96^{-/-} and DNAM-1^{-/-}CD96^{-/-} mice were injected subcutaneously on the right flank with various doses of MCA (5-400 µg, e.g. 100 µg MCA) and were monitored over time for fibrosarcoma formation. In addition, some mice were treated with control antibody, depleted of NK cells by treatment with anti-asialoGM1 (Wako Chemicals; 100 µg injected i.p. at day -1, 0 and then weekly until week 8), neutralized for IFN-γ (H22, 250 µg injected i.p. at day -1, 0 and then weekly until week 8), for CD155, for DNAM-1 or CD96.

### Dendritic cells (BMDC): NK cell coculture assays

BMDC were generated as described previously. Briefly, we harvested bone marrow cells from the femur and tibia of mice and cultured them in DMEM supplemented with 10% FCS, L-Glutamine, Penicillin/Streptomycin, Non-Essential Amino Acids, Sodium Pyruvate, β-2-mercaptoethanol and 250 ng/ml GM-CSF (eBioscience) for 6 days. WT or CD96^{-/-} NK cells were harvested from the spleens and FACS sorted to purity by staining with NK1.1 (PK136) and TCRβ (H57-597) and CD3 (17A2) antibodies. NK cells were harvested on the day of the assay. For assay set up, 5 × 10⁴ BMDM were plated in 96 well U bottom plates. NK cells were then added to the BMDM at varying titrations (2:1, 1:1, 0.5:1, and 0.25:1). BMDM only and NK only were always included in the assay as controls. Once all cells were plated, each well was filled with the appropriate amount of media to yield equivalent volumes between wells. 100 ng/ml of LPS was then added to the wells for 2 h, followed by 5 mM purified ATP (Sigma) for 30 mins. This was performed at 37°C in 5% CO₂. LPS only and ATP only controls were also included in the assay as controls. After 30 mins with ATP, supernatants were harvested and stored at -20°C until analysed.

### ⁵¹Cr Cytotoxicity Assays

Standard ⁵¹Cr cytotoxicity assays were used to analyse the ability of WT and CD96^{-/-} NK cells to kill targets. Briefly 20,000 targets labeled with 100 µCi of ⁵¹Cr were added to V bottom plates and NK cells were then added to the targets at defined effector to target ratios. After 4 h at 37°C in 5% CO₂, supernatants were harvested, and the level of ⁵¹Cr was quantified by a gamma counter (Wallac Wizard). Percentage specific killing was determined using the formula (Sample Cr release-Spontaneous Cr release)/(Total Cr release-Spontaneous Cr release) × 100.

### Cytokine Detection

All cytokine detection in serum or supernatants except IL-18 was achieved by utilising Cytometric Bead Array (CBA) technology (BD Biosciences). Acquisition was completed using a Canto II or LSRII Flow Cytometric Analyser (BD Biosciences). Analysis was performed using the FCAP array software. IL-18 was detected by an ELISA according to manufacturer's instructions (MRL). For intracellular cytokine detection, isolated lymphocytes were obtained from the liver, stained for surface markers, fixed and permeabilised (BD Biosciences), and stained with an anti-IFN-γ antibody (XMG1.2).

### Flow Cytometry Analysis and Sorting

Analysis of Immune Cell Homeostasis and CD96/CD155 expression: Various organs (lymph node, lung, spleen, bone marrow, and liver) were processed into single lymphocyte suspensions that included red blood cell lysis. Between 1 × 10⁶ and 5 × 10⁶ cells were initially subject to incubation with 2.4G2 to block non-specific Fc antibody binding before specific antibodies were utilised. To analyse NK cell homeostasis and IFN-γ production, the following antibodies were used: anti mouse-NK1.1, -TCRβ, -CD27 (LG.7F9), -CD11b (M1/70), and -IFN-γ. For T cells: anti mouse- TCRβ, -CD8 (53-6.7), and -CD4 (RM4-5). For B cells: anti mouse -B220 (RA3-6B2), -CD19 (1D3). For NKT cells: mouse CD1d tetramer loaded with α-galactosylceramide (kindly provided by Professor Dale Godfrey, University of Melbourne), anti mouse- TCRβ or -CD3, -CD4, and -NK1.1. For macrophages: anti mouse- F4/80 (BM8) and -CD11b. For neutrophils: anti mouse- Ly6G (1A8) and - CD11b. For conventional DC: anti mouse- MHC II (M5/114.15.2) and -CD11c (N418). For γδ T cells: anti mouse -γδ TCR (GL3) and -CD3. To analyse CD96 and CD155 expression, the specific cell type of interest was gated upon using the above antibody cocktails along with anti mouse- CD96 (3.3.3) or anti mouse- CD155 (4.24.3). Acquisition was performed using an LSR II, or Canto II flow cytometric analyser (BD Biosciences). Analysis was achieved using Flowjo (Treestar).

### Cell Sorting

Naive NK cells and macrophages from the spleen were prepared and stained for as described above. These cells were then sorted to purity using an Aria II FACS sorter (BD Biosciences).

### Statistical Analysis

Statistical analysis was achieved using Graphpad Prism Software. Data was considered to be statistically significant where the p value was equal to or less than 0.05. Statistical tests used were the unpaired Student's t test, Mann Whitney t test, and the Mantel-Cox test for survival. The appropriate test used is defined in the Figure legends.

### Results

CD96 competes with DNAM-1 for CD155 binding (Figure 1) and CD96 engagement by CD155 down-regulates NK cell production of IFNγ (Figure 2). CD96 limits NK cell-dependent tumor immunosurveillance in MCA-treated mice and promotes experimental B16F10 lung metastasis (Figure 3).

The data in Figure 4 show that anti-CD96 mAb has single agent activity (i.e without anti-PD1 treatment) while also enhancing the anti-tumor responses of anti-PD1. Anti-CD96 mAb treatment also enhances anti-tumor responses generated by Doxorubicin (DOX) chemotherapy (Figures 5 & 7) which is consistent with Figure 6 where enhanced anti-tumor responses to Doxorubicin (DOX) chemotherapy were observed in host with CD96 deficiency. Referring to Figures 8 & 9, given early or late, anti-CD96 mAb enhances anti-tumor responses generated by anti-PD-1 and anti-CTLA-4 mAbs and shows a particularly strong synergy with anti-PD-1.

The effect of CD96 in promotion of tumour metastasis was also investigated. In Figure 10, host CD96 promoted 3LL lung metastasis Figure 11 shows that anti-CD96 mAb suppresses B16F10 lung metastasis, alone and in combination with a T cell checkpoint blockade. In Figure 12, anti-CD96 mAb enhances anti-tumor responses generated by anti-PD-1 and anti-CTLA-4 mAbs against MC38 colon tumors and shows a particularly strong synergy with anti-PD-1.

### Example 2

### Screening assays for identifying anti-CD96 antibodies

### Introduction

The following assays may be used to identify antibodies useful in the invention. The first assay would be used to identify human antibodies capable of blocking or inhibiting binding between human CD96 and human CD155. The second assay may be used to test whether or not the identified antibodies cause antibody-dependent cell-mediated cytotoxicity (ADCC). The third assay can then be applied to lead candidates and involves determining whether or not a human CD96 antibody can modulate human lymphocyte effector function.

### Materials and Methods

### Assay 1: CD96 binding to CD155

The ability of candidate anti-CD96 antibodies to prevent the binding of CD155 to the cell surface of CD96 expressing cells (such as NK cells) will be tested as follows. Recombinant Human CD155 fused to the C terminal Fc region of human IgG1 (such as CD155-Fc available from Sino Biological) will be labeled with a fluorophore such as Alexa Fluor 647 (AF647) using Zenon Human IgG Labeling kit (Molecular Probe) accordingly to the manufacturer's instructions. NK cells or other CD96-expressing cells freshly isolated from the peripheral blood of healthy donors will be incubated with AF647 labeled CD155-Fc in the presence of anti-CD96 or control Ig at different concentrations ( The cells will be harvested and the cell surface binding of AF647-CD155-Fc will be tested by flow cytometry). Antibodies that prevent binding of CD155 cells to CD96-expressing cells will be identified by their ability to block binding of CD155-Fc to CD96-expressing cells.

### Assay 2: ADCC assay

The survival of immune cells (such as NK cells and/or T cells) in the presence of anti-CD96 antibodies will be analyzed as follows. The peripheral blood immune cells from healthy donors will be isolated by Ficoll gradient separation. Immune cells will be plated in 96 well plates in the presence of human IL-2 at an appropriate dosage and increasing concentrations of anti-CD96 mAbs. The survival as well as the percentages of CD96 expressing cells (such as NK cells and/or T cells) will be analyzed over time by flow cytometry. A non-limiting example of a suitable commercially available kit for this assay is the Annexin V Apoptosis Detection Kit.

### Assay 3: Assay for modulation of human leukocyte effector function by human CD96 antibodies

Fresh blood samples will be collected from healthy donors. Peripheral blood mononuclear cells (PBMC) will be prepared on a Ficoll-Paque density gradient by centrifugation. Highly pure CD3-CD56+ NK cells will be obtained from PBMC by magnetically activated cell sorting. To analyze the ability of CD96 to impact human NK cell production of IFN-γ, 96 well U bottom plates will be coated overnight at 4°C with recombinant Human CD155-Fc chimera (Sino Biological Inc.; 0.25 µg/ well) or with non-relevant human IgG1 antibodies. Freshly purified human NK cells will then be plated in complete RMPI media supplemented with Human IL-12, IL-18 and optionally IL-15 for 24 h and the intracellular content and the level of IFN-γ in the supernatant will be analysed in the different cultures. Alternatively, human NK cells will be stimulated for 24 h in wells coated with anti-NKG2D, anti-NKp46, anti-NKp30 or anti-CD16 antibodies to analyze the ability of CD96 signalling to interact with other NK cells receptors. The anti-human CD96 antibodies to be tested or control antibodies will be added to the cultures prior to the cytokines or antibodies above to confirm the ability of these test anti-human CD96 antibodies to enhance the IFNγ production of the human NK cells. Statistical increases in IFNγ production above the control would be considered significant.

### Example 3

### Assay for modulation of mouse NK cell function by anti-CD96 antibodies

Additional anti-mouse CD96 antibodies would also be screened for the ability to modulate CD96 signalling activity. To analyse mouse NK cell production of IFN-γ, 96 well U bottom plates would be coated overnight at 4°C with recombinant mouse CD155-Fc chimera (Sino Biological Inc.; 0.25 µg/ well) or with non-relevant human IgG1 antibodies. After three washes with PBS, freshly purified NK cells from the indicated mouse strains would be plated in complete RMPI media supplemented with mouse IL-12 (ebiosciences; 25-100 pg/ml) and mouse IL-18 (R&D; 50 ng/ml) for 24 h. Alternatively, IL-2-activatedNK cells would be stimulated for 6 h in wells coated with anti-NK1.1 (PK136; 0.125 µg/ well). At different time points, anti-mouse CD96 antibodies (50-200 µg/ml) or control antibodies will be added to determine whether these enhance NK cell IFN-g production as measured by CBA analysis.

### Example 4

### Production of mouse and human anti-CD96 antibodies

Human CD96 is a transmembrane protein that exists in two isoforms. Isoform 1 has been detected in acute myeloid leukaemia and includes additional amino acids compared with isoform 2. Isoform 2 is the common form in humans and the predicted domain architecture of isoform 2 has three (3) external immunoglobulin-like domains (domains 1, 2 and 3), as listed in Table 1. Antibodies against isoform 2 are preferred for use in the present invention. The nucleic and amino acid sequences of isoform 2 are given in the NCBI consensus sequence number CCDS2958.1 (SEQ ID NOS: 1 and 2 respectively).
SEQ ID NO:1. Human CD96 CDNA isoform 2. 
ATGGAGAAAAAATGGAAATACTGTGCTGTCTATTACATCATCCAGATACATTTTGTCAAGGGAGTTTG
GGAAAAAACAGTCAACACAGAAGAAAATGTTTATGCTACACTTGGCTCTGATGTCAACCTGACCTGCC
AAACACAGACAGTAGGCTTCTTCGTGCAGATGCAATGGTCCAAGGTCACCAATAAGATAGACCTGATT
GCTGTCTATCATCCCCAATACGGCTTCTACTGTGCCTATGGGAGACCCTGTGAGTCACTTGTGACTTT
CACAGAAACTCCTGAGAATGGGTCAAAATGGACTCTGCACTTAAGGAATATGTCTTGTTCAGTCAGTG
GAAGGTACGAGTGTATGCTTGTTCTGTATCCAGAGGGCATTCAGACTAAAATCTACAACCTTCTCATT
CAGACACACGTTACAGCAGATGAATGGAACAGCAACCATACGATAGAAATAGAGATAAATCAGACTCT
GGAAATACCATGCTTTCAAAATAGCTCCTCAAAAATTTCATCTGAGTTCACCTATGCATGGTCGGTGG
AGGATAATGGAACTCAGGAAACACTTATCTCCCAAAATCACCTCATCAGCAATTCCACATTACTTAAA
GATAGAGTCAAGCTTGGTACAGACTACAGACTCCACCTCTCTCCAGTCCAAATCTTCGATGATGGGCG
GAAGTTCTCTTGCCACATTAGAGTCGGTCCTAACAAAATCTTGAGGAGCTCCACCACAGTCAAGGTTT
TTGCTAAACCAGAAATCCCTGTGATTGTGGAAAATAACTCCACGGATGTCTTGGTAGAGAGAAGATTT
ACCTGCTTACTAAAGAATGTATTTCCCAAAGCAAATATCACATGGTTTATAGATGGAAGTTTTCTTCA
TGATGAAAAAGAAGGAATATATATTACTAATGAAGAGAGAAAAGGCAAAGATGGATTTTTGGAACTGA
AGTCTGTTTTAACAAGGGTACATAGTAATAAACCAGCCCAATCAGACAACTTGACCATTTGGTGTATG
GCTCTGTCTCCAGTCCCAGGAAATAAAGTGTGGAACATCTCATCAGAAAAGATCACTTTTCTCTTAGG
TTCTGAAATTTCCTCAACAGACCCTCCACTGAGTGTTACAGAATCTACCCTTGACACCCAACCTTCTC
CAGCCAGCAGTGTATCTCCTGCAAGATATCCAGCTACATCTTCAGTGACCCTTGTAGATGTGAGTGCC
TTGAGGCCAAACACCACTCCTCAACCCAGCAATTCCAGTATGACTACCCGAGGCTTCAACTATCCCTG
GACCTCCAGTGGGACAGATACCAAAAAATCAGTTTCACGGATACCTAGTGAAACATACAGTTCATCCC
CGTCAGGTGCAGGCTCAACACTTCATGACAATGTCTTTACCAGCACAGCCAGAGCATTTTCAGAAGTC
CCCACAACTGCCAATGGATCTACGAAAACTAATCACGTCCATATCACTGGTATTGTGGTCAATAAGCC
CAAAGATGGAATGTCCTGGCCAGTGATTGTAGCAGCTTTACTCTTTTGCTGCATGATATTGTTTGGTC
TTGGAGTGAGAAAATGGTGTCAGTACCAAAAAGAAATAATGGAAAGACCTCCACCTTTCAAGCCACCA
CCACCTCCCATCAAGTACACTTGCATTCAAGAGCCCAACGAAAGTGATCTGCCTTATCATGAGATGGA
GACCCTCTAG
SEQ ID NO:2. Human CD96 protein sequence isoform 2

MEKKWKYCAVYYIIQIHFVKGVWEKTVNTEENVYATLGSDVNLTCQTQTVGFFVQMQWSKVTNKIDLI
AVYHPQYGFYCAYGRPCESLVTFTETPENGSKWTLHLRNMSCSVSGRYECMLVLYPEGIQTKIYNLLI
QTHVTADEWNSNHTIEIEINQTLEIPCFQNSSSKISSEFTYAWSVEDNGTQETLISQNHLISNSTLLK
DRVKLGTDYRLHLSPVQIFDDGRKFSCHIRVGPNKILRSSTTVKVFAKPEIPVIVENNSTDVLVERRF
TCLLKNVFPKANITWFIDGSFLHDEKEGIYITNEERKGKDGFLELKSVLTRVHSNKPAQSDNLTIWCM
ALSPVPGNKVWNISSEKITFLLGSEISSTDPPLSVTESTLDTQPSPASSVSPARYPATSSVTLVDVSA
LRPNTTPQPSNSSMTTRGFNYPWTSSGTDTKKSVSRIPSETYSSSPSGAGSTLHDNVFTSTARAFSEV
PTTANGSTKTNHVHITGIVVNKPKDGMSWPVIVAALLFCCMILFGLGVRKWCQYQKEIMERPPPFKPP
PPPIKYTCIQEPNESDLPYHEMETL

The Mouse CD96 protein is also a transmembrane protein but only a single transcript/isoform is known in the mouse as shown in SEQ ID NOs. 3 and 4.
SEQ ID NO: 3. Mouse cDNA 
ATGGGGAGAAAATGGACATATTGTGTTGTTTACACCATCATCCAGATACAGTTTTCAGGGGAGTGTG
GGAAGAGCTATTCAATGTTGGAGATGACGTGTATGCTCTACCTGGTTCTGACATCAACTTGACCTGCC
AAACAAAGGAGAAAAACTTCTTGGTACAGATGCAGTGGTCCAAGGTCACAGATAAGAATGACATGATT
GCTCTTTATCATCCCCAATATGGCCTCTACTGTGGGCAGGAGCATGCCTGTGAGTCACAAGTGGCTGC
CACAGAAACTGAGAAGGGTGTAACAAATTGGACTCTGTACTTAAGGAATATCTCTTCTGCCCTGGGTG
GAAAGTATGAGTGCATCTTTACTCTGTATCCAGAAGGCATCAAGACTACAGTCTACAACCTCATTGTG
GAACCCTATACACAAGATGAACACAACTATACAATAGAAATAGAGACAAATCGGACTCTGGAAATACC
ATGCTTTCAAAATACCTCCTCAGAAATTCCACCTAGGTTCACCTTTTCATGGTTGGTGGAGAAAGATG
GAGTGGAAGAAGTTCTCTTCACCCACCATCACCACGTCAACAATTCCACATCATTTAAAGGCAGAATC
AGGCTGGGTGGAGACTATAGACTCCACCTCTCCCCAGTCCAAATCCAAGACGATGGCAGGACATTCTC
TTGCCATCTGACTGTCAATCCTCTCAAAGCCTGGAAGATGTCCACCACAGTCAAGGTTTTTGCTAAAC
CAGAAATCCTCATGACTGTGGAAAACAGCACCATGGATGTCTTAGGAGAGAGAGTATTTACCTGCCTA
CTGAAGAATGTGTTCCCCAAGGCAAATATCACCTGGTTTATAGACGGAAGATTTCTTCAAGGCAACGA
AGAAGGAATATACATTACAAATGAAGAGAAGAATTGCAGTAGCGGATTTTGGGAACTGAAGTCAGTTT
TAACAAGGATGCACAGTGGACCATCCCAATCAAACAACATGACAGCTTGGTGTATGGCTCTGTCTCCA
GGCCCCAGAAATAAAATGTGGAATACTTCATCACAACCCATCACGGTTTCCTTTGATTCGGTGATAGC
CCCAACGAAACATCTACCCACTGTGACAGGTTCTACCCTGGGTACACAACCTTTTCAGATGCTGGAG
TATCTCCTACAGGGTATCTAGCTACACCTTCAGTGACAATTGTAGATGAAAATGGGTTGACACCAGAT
GCAACTCCTCAAACCAGCAATTCCAGCATGACTACTAAAGATGGCAACTATTTGGAAGCCTCCAGTGG
GACAGATGCCAAGAACTCCTCAAGAGCTGCTGCTTCTTCTAAAAGTGGATCTTGGCCTTTTCCTTTCA
CTTCTCCTCCAGAATGGCACTCACTGCCTGGTACCTCCACTGGACCCCAAGAACCAGACTCCCCAGTT
TCATGGATACCCAGTGAAGTACACACTTCAGCCCCTTTGGACGCCAGCTTAGCTCCTCATGATACCAT
CATCAGTACAACCACAGAATTTCCAAATGTCCTCACAACTGCAAATGGAACTACTAAAATTGACCATG
GACCTATCACCAGTATCATAGTTAATCAACCCAGTGATGGAATGTCCTGGCCTGTGCTTGTCGCGGCT
TTGCTCTTTTTCTGCACACTATTGTTTGGGCTTGGAGTAAGAAAATGGTATCGGTATCAAAATGAAAT
CATGGAGAGACCCCCACCTTTCAAGCCACCACCACCTCCCATCAAGTACACGTATATTCAAGAACCCA
TTGGATGCGACCTGTGTTGTCATGAGATGGAGGTCCTCTAA
SEQ ID NO:4. Mouse CD96 amino acid sequence

MGRKWTYCVVYTIIQIQFFRGVWEELFNVGDDVYALPGSDINLTCQTKEKNFLVQMQWSKVTDKNDMI
ALYHPQYGLYCGQEHACESQVAATETEKGVTNWTLYLRNISSALGGKYECIFTLYPEGIKTTVYNLIV
EPYTQDEHNYTIEIETNRTLEIPCFQNTSSEIPPRFTFSWLVEKDGVEEVLFTHHHHVNNSTSFKGRI
RLGGDYRLHLSPVQIQDDGRTFSCHLTVNPLKAWKMSTTVKVFAKPEILMTVENSTMDVLGERVFTCL
LKNVFPKANITWFIDGRFLQGNEEGIYITNEEKNCSSGFWELKSVLTRMHSGPSQSNNMTAWCMALSP
GPRNKMWNTSSQPITVSFDSVIAPTKHLPTVTGSTLGTQPFSDAGVSPTGYLATPSVTIVDENGLTPD
ATPQTSNSSMTTKDGNYLEASSGTDAKNSSRAAASSKSGSWPFPFTSPPEWHSLPGTSTGPQEPDSPV
SWIPSEVHTSAPLDASLAPHDTIISTTTEFPNVLTTANGTTKIDHGPITSIIVNQPSDGMSWPVLVAA
LLFFCTLLFGLGVRKWYRYQNEIMERPPPFKPPPPPIKYTYIQEPIGCDLCCHEMEVL

**Table 1: External immunoglobulin like domains of CD96 from Interpro predictions**

| | **Human CD96 isoform 2 residue numbers** | **Mouse CD96 residue numbers** |
|---|---|---|
| Domain 1 | 30-137 | 30-137 |
| Domain 2 | 148-250 | 145-247 |
| Domain 3 | 253-359 | 250-355 |

The external domains of the mouse and human CD96 proteins will be cloned into appropriate expression constructs for expression in mammalian cells such as human embryonic kidney cells. Suitable expression constructs typically include a CMV promoter to drive expression of the CD96 gene fragment. Following transfection into mammalian cells the protein will be expressed under suitable culture conditions before being purified prior to antibody production.

Four CD96 knockout mice will be immunized with the mouse CD96 external domain protein and likewise four CD96 knockout mice will be immunized with the purified human CD96 external domain protein. Immunisations will be made at approximately three times at four week intervals. The mice will be bled 10-12 days following the third immunization and the sera titrated on the screening antigens by ELISA. The mice with the highest antibody titres would be used for fusion, otherwise if mice have not responded adequately further immunisations would be undertaken. Selected hybridomas will be cloned and mAbs purified from each clone before the individual human or mouse mAbs are screened using the screening assays of Examples 2 or 3 respectively. Isotyping of clones would optionally be undertaken in order to identify antibodies that are less likely or unable to induce ADCC, such as IgG2 and IgG4 antibodies. Approximately 20 antibodies against human CD96 and 20 antibodies against mouse CD96 would be obtained as listed in Table 1 of Example 4.

### Example 5

### Screening of mouse and human anti-CD96 antibodies

Approximately 20 each of anti-mouse CD96 and anti-human CD96 monoclonal antibodies would be obtained as described in Example 4. The anti-human CD96 antibodies would be screened for the ability to modulate CD96 signalling activity using the Human NK cell assays described in Example 2. An additional four commercially available anti-human CD96 antibodies (1C8, NK92.39, 3H8, MAA6359) will also be screened for the ability to modulate CD96 signalling. Anti-mouse CD96 mAbs would be also screened for their ability to modulate NK cell function as described in Example 3.

As can occur with antibodies, not all antibodies are expected to have a useful effect on a given target. Accordingly, CD96 signalling for each antibody will be assessed using the human or mouse NK cell assays to determine which antibodies have an effect upon CD96 signalling.

### Preliminary results using the human NK cell assay

Using the human NK cell assay described in Example 2, the NK92.39 human CD96 mAb was found to increase the levels of IFN-γ in human NK cells as shown in Figure 14A and 14B. This result indicates that antibodies against the human CD96 receptor can be effective in increasing IFN-γproduction in human NK cells.

### Example 6

### Anti-mouse CD96 antibody testing in cancer models

Anti-mouse CD96 antibodies that are found to be active in modulating CD96 signalling will be tested for their ability to relieve immune inhibition, reduce CD96 activity and/or enhance or restore immune surveillance in mouse cancer models.

Approximately 10 active anti-mouse CD96 antibodies will be individually tested in approximately 5-7 cancer models using *In vivo* tumor challenges, the same or similar to those used in Example 1.

The efficacy of each CD96 antibody against a respective tumour and/or in a tumour model may vary and some may be more responsive to treatment than others. Non-limiting examples of tumours and tumour models to be tested may include breast, prostate, lung, melanoma, colorectal, pancreatic, endometrial, kidney, bowel, gastric, oesophageal, leukaemia, lymphoma, ovarian, bladder and brain cancers including primary tumours and/or metastases of the aforementioned cancers..

### Example 7

### Human CD96-antibody binding studies

A subset of anti-human CD96 antibodies will be examined further to determine which domains of CD96 are bound by effective CD96 antibodies. As discussed in Example 3 above, isoform 2 of CD96 includes three external domains (Domains 1, 2 and 3).

Antibodies will be bound to the CD96 protein and the specific CD96 protein residues bound will be determined using hydrogen/deuterium exchange and mass spectrometry.^{23,24} Alternatively, other methods may be employed to probe antibody-CD96 binding sites such as X-ray crystallography, site directed mutagenesis or other methods known in the art.

It is anticipated that effective antibodies may bind one or multiple external domains of the CD96 protein. For example anti-CD96 antibodies that modulate human NK cell function may bind any of the following combinations of external CD96 domains, with each possible single or combination binding domain shown in brackets: (1), (2), (3), (1,2), (1,3), (2,3), (1,2,3).

### Example 8

### CD96 roles in both NK and T cell function

Natural killer (NK) cells are innate lymphocytes which may be critical to limit early tumour growth and metastasis while T cells may be more important in the control of established and primary tumours. CD96 is a checkpoint immunomodulator that can affect both NK and T cell function.

Primary tumors were used to examine the role of CD96 in T cells. The AT3-OVA^{dim} model was used where CD8⁺T effector cells are known to naturally control tumor growth. AT3-OVA^{dim} mammary carcinoma (1× 10⁶ cells) were injected subcutaneously. Mice were then monitored for tumor growth and measurements made with a caliper square as the product of two perpendicular diameters (mm²).

Anti-CD96 treatment greatly reduced the rate of tumour growth and this beneficial effect could be removed by depletion of CD4 and CD8 T cells using anti CD4/CD8 antibody (Figure 13). This demonstrates that anti-CD96 mAb critically required CD8⁺ T cells for full anti-tumour activity in this particular tumour model.

### Example 9

### Loss of CD96 from the NK cell surface after aCD96 mAb binding

The mechanistic/signaling effects that may occur upon antibody binding to CD96 may also reveal functional information about the effects of CD96-CD155 binding. To further investigate this, total NK cells were purified from human peripheral blood mononuclear cells (PBMCs) by negative selection using human NK cell isolation kit (Miltenyi Biotec.). Isolated NK cells were then labeled with carboxyfluorescein diacetate succinmidyl ester (CFSE; Biolegend) to measurecellular proliferation. CFSE-labeled NK cells were plated in 96 well U-bottom plate at 5 × 10⁴ cells/well and stimulated with recombinant IL-2 at indicated concentrations (10 units/ml and 25 units/ml), in the presence of control IgG or anti-human CD96 mAb (clone NK92-39) at 30 µg/ml. NK cells were assessed for changes in proliferation or the presence/absence of surface CD96 at day 3 and 6 using BD FACS Canto II (BD Biosciences) and analysis was carried out using FlowJo (Tree Star) (Figures 15A and 15B). Anti-CD96 binding to CD96 had no effect on NK cell proliferation but appeared to greatly reduce the level of CD96 on the cell surface by day 6, either by internalization of CD96 following mAb binding or possibly via a reduction in CD96 expression.

Throughout the specification the aim has been to describe the preferred embodiments of the invention without limiting the invention to any one embodiment or specific collection of features. It will therefore be appreciated by those of skill in the art that, in light of the instant disclosure, various modifications and changes can be made in the particular embodiments exemplified without departing from the scope of the present invention.

All computer programs, algorithms, patent and scientific literature referred to herein is incorporated herein by reference.

### REFERENCES

*1.* Vivier, E., Tomasello, E., Baratin, M., Walzer, T. & Ugolini, S. Functions of natural killer cells. Nature immunology 9, 503-510 (2008*).*
*2.* Lanier, L.L. Up on the tightrope: natural killer cell activation and inhibition. Nature immunology 9, 495-502 (2008*).*
*3.* Chan, C.J., Smyth, M.J. & Martinet, L. Molecular mechanisms of natural killer cell activation in response to cellular stress. Cell death and differentiation (2013*).*
*4.* Raulet, D.H. & Vance, R.E. Self-tolerance of natural killer cells. Nature reviews 6, 520-531 (2006*).*
*5.* Fuchs, A. & Colonna, M. The role of NK cell recognition of nectin and nectin-like proteins in tumor immunosurveillance. Seminars in cancer biology 16, 359-366 (2006*).*
*6.* Shibuya, A., et al. DNAM-1, a novel adhesion molecule involved in the cytolytic function of T lymphocytes. Immunity 4, 573-581 (1996*).*
*7.* Wang, P.L., O'Farrell, S., Clayberger, C. & Krensky, A.M. Identification and molecular cloning of tactile. A novel human T cell activation antigen that is a member of the Ig gene superfamily. J Immunol 148, 2600-2608 (1992*).*
*8.* Yu, X, et al. The surface protein TIGIT suppresses T cell activation by promoting the generation of mature immunoregulatory dendritic cells. Nature immunology 10, 48-57 (2009*).*
*9.* Boles, K. S., et al. A novel molecular interaction for the adhesion of follicular CD4 T cells to follicular DC. European journal of immunology 39, 695-703 (2009*).*
*10.* Kennedy, J., et al. A molecular analysis of NKT cells: identification of a class-I restricted T cell-associated molecule (CRTAM). Journal of leukocyte biology 67, 725-734 (2000*).*
*11.* Bottino, C., et al. Identification of PVR (CD155) and Nectin-2 (CD112) as cell surface ligands for the human DNAM-1 (CD226) activating molecule. The Journal of experimental medicine 198, 557-567 (2003*).*
*12.* Lozano, E., Dominguez-Villar, M., Kuchroo, V. & Hafler, D.A. The TIGIT/CD226 axis regulates human T cell function. Journal of immunology 188, 3869-3875 (2012*).*
*13.* Lakshmikanth, T., et al. NCRs and DNAM-1 mediate NK cell recognition and lysis of human and mouse melanoma cell lines in vitro and in vivo. The Journal of clinical investigation 119, 1251-1263 (2009*).*
*14.* Chan, C.J., et al. DNAM-1/CD155 interactions promote cytokine and NK cell-mediated suppression of poorly immunogenic melanoma metastases. J Immunol 184, 902-911 (2010*).*
*15.* Gilfillan, S., et al. DNAM-1 promotes activation of cytotoxic lymphocytes by nonprofessional antigen-presenting cells and tumors. The Journal of experimental medicine 205, 2965-2973 (2008*).*
*16.* Iguchi-Manaka, A., et al. Accelerated tumor growth in mice deficient in DNAM-1 receptor. The Journal of experimental medicine 205, 2959-2964 (2008*).*
*17.* Stanietsky, N., et al. The interaction of TIGIT with PVR and PVRL2 inhibits human NK cell cytotoxicity. Proceedings of the National Academy of Sciences of the United States of America 106, 17858-17863 (2009*).*
*18.* Stanietsky, N., et al. Mouse TIGIT inhibits NK-cell cytotoxicity upon interaction with PVR. European journal of immunology (2013*).*
*19.* Liu, S., et al. Recruitment of Grb2 and SHIP1 by the ITT-like motif of TIGIT suppresses granule polarization and cytotoxicity of NK cells. Cell death and differentiation 20, 456-464 (2013*).*
*20.* Fuchs, A., Cella, M., Kondo, T. & Colonna, M. Paradoxic inhibition of human natural interferon-producing cells by the activating receptor NKp44. Blood 106, 2076-2082 (2005*).*
*21.* Seth, S., et al. The murine pan T cell marker CD96 is an adhesion receptor for CD155 and nectin-1. Biochemical and biophysical research communications 364, 959-965 (2007*).*
*22.* Fuchs, A., Cella, M., Giurisato, E., Shaw, A.S. & Colonna, M. Cutting edge: CD96 (tactile) promotes NK cell-target cell adhesion by interacting with the poliovirus receptor (CD155). J Immunol 172, 3994-3998 (2004*).*
*23.* Ahn, J., Skilton, J. & Yu, K. Hydrogen Deuterium Exchange Mass Spectrometry: An Emerging Biophysical Tool for Probing Protein Behavior and Higher-Order Structure. LCGC NORTH AMERICA VOLUME 31 NUMBER 6 (2013*).*
*24.* Percy, A. J., Rey, M., Burns, K. M. & Schriemer, D. C. Probing protein interactions with hydrogen/deuterium exchange and mass spectrometry A review. Analytica Chimica Acta 721 (2012) 7 21 (2012*).*

## Claims

1. A CD96-inhibitory agent for use in treatment or prevention of cancer in a human, by at least partly inhibiting or reducing CD96 activity in one or more cells of the human to thereby relieve immune inhibition and/or enhance or restore immune surveillance in the human, wherein the CD96-inhibitory agent is an antibody or antibody fragment that binds CD96.

2. The CD96-inhibitory agent for use according to Claim 1, wherein the CD96-inhibitory agent binds or interacts with only one external immunoglobulin-like domain of human CD96 isoform 2 (SEQ ID NO:2).

3. The CD96-inhibitory agent for use according to Claim 1, wherein the CD96-inhibitory agent binds or interacts with a plurality of external immunoglobulin-like domains of human CD96 isoform 2 (SEQ ID NO:2).

4. The CD96-inhibitory agent for use according to Claim 1, 2 or 3, which includes one or more other therapeutic agents.

5. The CD96-inhibitory agent for use according to Claim 4, wherein the one or more other therapeutic agents is a chemotherapeutic agent and one or more antibodies or antibody fragments that bind PD1 and/or CTLA4.

6. An *in vitro* method of screening, designing, engineering or otherwise producing a CD96-inhibitory agent, said method including the steps of identifying a candidate molecule that binds CD96 and determining that the candidate molecule is capable of at least partly inhibiting or reducing CD96 activity to thereby relieve immune inhibition and/or enhance or restore immune surveillance in a human, wherein the CD96-inhibitory agent is an anti-cancer agent and wherein the CD96-inhibitory agent is an antibody or antibody fragment.

7. The method of Claim 6, wherein the CD96-inhibitory agent binds or interacts with one or a plurality of external immunoglobulin-like domains of CD96.

8. The method of Claim 7, wherein the one or plurality of external immunoglobulin-like domains of CD96 are selected from the group consisting of: domain 1; domain 2; domain 3; domain 1 and domain 2; domain 1 and domain 3; domain 2 and domain 3; and domain 1, domain 2 and domain 3.

9. The method of Claim 6, 7 or 8, wherein the CD96-inhibitory agent binds or interacts with only one external immunoglobulin-like domain of human CD96 isoform 2 (SEQ ID NO:2).

10. The method of Claim 6, 7 or 8, wherein the CD96-inhibitory agent binds or interacts with a plurality of external immunoglobulin-like domains of human CD96 isoform 2 (SEQ ID NO: 2).

## Patentansprüche

1. CD96-hemmendes Mittel zur Verwendung bei der Behandlung oder Vorbeugung von Krebs bei einem Menschen, durch mindestens teilweises Hemmen oder Reduzieren von CD96-Aktivität in einer oder mehreren Zelle(n) des Menschen, um dadurch die Immunhemmung zu lindern und/oder die Immunüberwachung bei dem Menschen zu erhöhen oder wiederherzustellen, wobei das CD96-hemmende Mittel ein Antikörper oder Antikörperfragment ist, das CD96 bindet.

2. CD96-hemmendes Mittel zur Verwendung nach Anspruch 1, wobei das CD96-hemmende Mittel nur eine externe Immunglobulin-ähnliche Domäne der menschlichen CD96-Isoform 2 (SEQ ID NO: 2) bindet oder mit dieser interagiert.

3. CD96-hemmendes Mittel zur Verwendung nach Anspruch 1, wobei das CD96-hemmende Mittel eine Vielzahl von externen Immunglobulin-ähnlichen Domänen der menschlichen CD96-Isoform 2 (SEQ ID NO: 2) bindet oder mit diesen interagiert.

4. CD96-hemmendes Mittel zur Verwendung nach Anspruch 1, 2 oder 3, welches ein oder mehrere andere therapeutische(s) Mittel umfasst.

5. CD96-hemmendes Mittel zur Verwendung nach Anspruch 4, wobei das eine oder die mehreren anderen therapeutische(n) Mittel ein chemotherapeutisches Mittel und ein oder mehrere Antikörper oder Antikörperfragment(e), die PD1 und/oder CTLA4 binden, ist/ sind.

6. *In-vitro*-Verfahren zum Screening, Entwerfen, Konstruieren oder anderweitigem Herstellen eines CD96-hemmenden Mittels, wobei das Verfahren die Schritte des Identifizierens eines Kandidatenmoleküls, das CD96 bindet, und des Bestimmens, dass das Kandidatenmolekül in der Lage ist, mindestens teilweise CD96-Aktivität zu hemmen oder zu reduzieren, um dadurch die Immunhemmung zu lindern und/oder die Immunüberwachung bei einem Menschen zu erhöhen oder wiederherzustellen, umfasst, wobei das CD96-hemmende Mittel ein Antikrebsmittel ist und wobei das CD96-hemmende Mittel ein Antikörper oder Antikörperfragment ist.

7. Verfahren nach Anspruch 6, wobei das CD96-hemmende Mittel eine oder eine Vielzahl von externe(n) Immunglobulin-ähnliche(n) Domäne(n) von CD96 bindet oder mit dieser/n interagiert.

8. Verfahren nach Anspruch 7, wobei die eine oder die Vielzahl von externe(n) Immunglobulin-ähnliche(n) Domäne(n) von CD96 ausgewählt ist/ sind aus der Gruppe bestehend aus: Domäne 1; Domäne 2; Domäne 3; Domäne 1 und Domäne 2; Domäne 1 und Domäne 3; Domäne 2 und Domäne 3; und Domäne 1, Domäne 2 und Domäne 3.

9. Verfahren nach Anspruch 6, 7 oder 8, wobei das CD96-hemmende Mittel nur eine externe Immunglobulin-ähnliche Domäne der menschlichen CD96-Isoform 2 (SEQ ID NO: 2) bindet oder mit dieser interagiert.

10. Verfahren nach Anspruch 6, 7 oder 8, wobei das CD96-hemmende Mittel eine Vielzahl von externen Immunglobulin-ähnlichen Domänen der menschlichen CD96-Isoform 2 (SEQ ID NO: 2) bindet oder mit diesen interagiert.

## Revendications

1. Agent inhibiteur de CD96 pour utilisation dans le traitement ou la prévention du cancer chez un humain, en inhibant ou en réduisant au moins partiellement l'activité de CD96 dans une ou plusieurs cellules de l'humain pour ainsi soulager l'inhibition immunitaire et/ou améliorer ou restaurer la surveillance immunitaire chez l'humain, l'agent inhibiteur de CD96 étant un anticorps ou un fragment d'anticorps qui se lie à CD96.

2. Agent inhibiteur de CD96 pour utilisation selon la revendication 1, l'agent inhibiteur de CD96 se liant ou interagissant avec un seul domaine externe de type immunoglobuline de l'isoforme 2 de CD96 humain (SEQ ID NO : 2).

3. Agent inhibiteur de CD96 pour utilisation selon la revendication 1, l'agent inhibiteur de CD96 se liant ou interagissant avec une pluralité de domaines externes de type immunoglobuline de l'isoforme 2 de CD96 humain (SEQ ID NO : 2).

4. Agent inhibiteur de CD96 pour utilisation selon la revendication 1, 2 ou 3, qui comprend un ou plusieurs autres agents thérapeutiques.

5. Agent inhibiteur de CD96 pour utilisation selon la revendication 4, dans lequel les un ou plusieurs autres agents thérapeutiques sont un agent chimiothérapeutique et un ou plusieurs anticorps ou fragments d'anticorps qui se lient à PD1 et/ou CTLA4.

6. Procédé *in vitro* de criblage, de conception, d'ingénierie ou de production autre d'un agent inhibiteur de CD96, ledit procédé comprenant les étapes consistant à identifier une molécule candidate qui se lie à CD96 et à déterminer que la molécule candidate est capable d'inhiber ou de réduire au moins partiellement l'activité de CD96 pour ainsi soulager l'inhibition immunitaire et/ou améliorer ou restaurer la surveillance immunitaire chez un humain, dans lequel l'agent inhibiteur de CD96 est un agent anticancéreux et dans lequel l'agent inhibiteur de CD96 est un anticorps ou un fragment d'anticorps.

7. Procédé selon la revendication 6, dans lequel l'agent inhibiteur de CD96 se lie ou interagit avec un ou une pluralité de domaines externes de type immunoglobuline de CD96.

8. Procédé selon la revendication 7, dans lequel l'un ou la pluralité de domaines externes de type immunoglobuline de CD96 sont choisis dans le groupe constitué par : le domaine 1 ; le domaine 2 ; le domaine 3 ; le domaine 1 et le domaine 2 ; le domaine 1 et le domaine 3 ; le domaine 2 et le domaine 3 ; et le domaine 1, le domaine 2 et le domaine 3.

9. Procédé selon la revendication 6, 7 ou 8, dans lequel l'agent inhibiteur de CD96 se lie ou interagit avec un seul domaine externe de type immunoglobuline de l'isoforme 2 de CD96 humain (SEQ ID NO : 2)

10. Procédé selon la revendication 6, 7 ou 8, dans lequel l'agent inhibiteur de CD96 se lie ou interagit avec une pluralité de domaines externes de type immunoglobuline de l'isoforme 2 de CD96 humain (SEQ ID NO : 2).
